(19) Europäisches Patentamt
European Patent Office
Office européen des brevets

(11) EP 0 713 876 A1

(12) **EUROPEAN PATENT APPLICATION**

(43) Date of publication:
**29.05.1996 Bulletin 1996/22**

(21) Application number: **95118477.9**

(22) Date of filing: **23.11.1995**

(51) Int. Cl.[6]: **C07D 487/04**, A61K 31/53
// (C07D487/04, 253:00, 235:00), (C07D487/04, 253:00, 209:00)

(84) Designated Contracting States:
**DE FR GB IT**

(30) Priority: **24.11.1994 JP 312354/94**

(71) Applicant: **Wakamoto Pharmaceutical Co., Ltd.**
**Chuo-ku, Tokyo 103 (JP)**

(72) Inventors:
- **Kawano, Katsuhiro, c/o Wakamoto Pharmaceutical Co. Tokyo 103 (JP)**
- **Akiba, Kiyoshi, c/o Wakamoto Pharmaceutical Co., Tokyo 103 (JP)**
- **Toyofuku, Hatsunori, c/o Wakamoto Pharmaceutical Tokyo 103 (JP)**
- **Agata, Mitsuzi, c/o Wakamoto Pharmaceutical Co., Tokyo 103 (JP)**
- **Ohmura, Takeo, c/o Wakamoto Pharmaceutical Co., Tokyo 103 (JP)**
- **Maeda, Makoto, c/o Wakamoto Pharmaceutical Co., Tokyo 103 (JP)**

(74) Representative: **VOSSIUS & PARTNER**
**Siebertstrasse 4**
**81675 München (DE)**

(54) **Triazine derivative, chymase activity inhibitor and nitric oxide production inhibitor**

(57) The present invention provides novel triazine derivatives which inhibit chymase activity and nitric oxide production. The triazine derivatives are useful for the prevention and treatment of bronchial asthma, allergic rhinitis and hives by inhibiting chymase activity, and are useful for the prevention and treatment of cardiopathy and cerebrovascular disease, ishemic heart disease, septic shock, ache, rheumatic fever, arthritis, asthma, immunodeficiency, viral or non-viral infections, autoimmune diseases and cancer by inhibiting nitric oxide production.

EP 0 713 876 A1

## Description

The present invention relates to novel triazine derivatives which are useful as a chymase inhibitor and a nitric oxide production inhibitor.

Chymase is a chymotrypsin-like serine protease which has been found in Mast Cell (MC)-secretory granules (Katunuma N. et al., Eur. J. Biochem., 52, 37-50, (1975)), and it functions generally during inflammation as described below: it has been found that release of histamine granules mediated by the IgE receptor is inhibited by inhibiting chymase activity which is localized in MC (Kido H. et al, Biochem. Int., 10, 863-871, (1985); Kato Y. et al., J. Biochem., 103, 820-822, (1988); Dietze S. C., Biol. Chem. Hoppe-Seyler, 371, Suppl. 75-79, (1990)); it has been found that a chymase-like enzyme is localized in eosinophil granules, and it relates to mechanism on degranulation (Kawaji Y. et al., Allergy, 42, 1591-1599, (1993) ); on the other hand, chymase which is released extracellularly in association with degranulation rapidly bonds to neighboring cell membranes (Schwartz L. B. et al., J. Immunol., 126, 2071-2078, (1981)), and cuts extracellular substrate of type IV collagen (Sage H. et al., J. Biol. Chem., 254, 9893-9900, (1979)) and of fibronectin (Vartio T. et al., J. Biol. Chem., 256, 471-477, (1981)), and enhances vascular permeability together with histamine which has been released by degranulation with chymase (Seppa H., Inflammation, 4, 1-8, (1980)); at this time, chymase enhances histamine activity (Rubinstein I. et al., J. Clin. Invest., 86, 555-559, (1990)), and also produces histamine-releasing peptides from serum albumin (Cochrane D. E. et al., Peptides, 14, 117-123, (1993); chymase further cuts intracellular substrates such as collagen and proteoglycan (Seppa H. et al., Acta. Histochem., 64, 64-70 (1979); Briggaman R. A. et al., J. Exp. Med., 160, 1027-1042, (1984)); chymase decomposes restrictively IgE to form leukocyte migration factor (Katunuma N. et al, In Weber G "Advances in Enzyme Regulation", Oxford, Pergamon Press 241-255, (1986)) and converts the precursor of interleukin-1-β which is one of the inflammatory cytokines into an active substance (Mizutani H. et al., J. Exp. Med. 821-825, (1991)). Thus chymase enhances local mobilization of humoral substances mediated by venula, and further enhances infiltration of the humoral substances into tissues.

Chymase also relates to the metabolism of vascactive peptides.

For example, chymase is considered an Angiotensin II (Ang II) producing enzyme other than Angiotensin converting enzyme (Okunishi H. et al., J. Hypertens., 2, 277-284, (1984); Urata H. et al., J. Biol. Chem., 265, 22348-22357, (1990)), and it is indicated that chymase plays a role to establish endosporium hyperplasia lesions after angiopathy mediated by Ang II (Shiota N. et al., FEBS Letter, 323, 239-242, (1993)). Chymase also enhances secretion of adenocyte (Sommerhoff C. P. et al., J Immunol., 142, 2450-2456, (1989)). Further it is indicated that chymase is related to the accumulation of β-amyloid in Alzheimer's disease (Nelson R. B. et al., J. Neurochem., 61, 567-577, (1993)).

As mentioned above, it is considered that chymase is deeply Involved in the formation of diseases of allergic inflammation, and it is expected that the inhibition of this enzyme is effective in treating and preventing allergic diseases such as bronchial asthma, allergic rhinitis and hives. There have been already reports of some chymotrypsin-like enzyme inhibitors and serine protease inhibitors as a substance having chymase inhibitory activity. As naturally occurring substances, examples thereof include $\alpha_1$-antichymotrypsin which is a serum protein protease inhibitor (Schechter N. M. et al., J. Biol. Chem., 268, 23626-23633, (1993)), lima seed trypsin inhibitor originated from a plant (Schechter N. M. et al., J. Biol. Chem., 258, 2973-2978, (1983)) and chymostatin originated from microoraganisms (Okuno-Kuneda S. et al., Biochem. Pharmacol., 29, 1715-1722, (1980)). As non-naturally occurring substances, there are reported peptidic chloromethylketone derivatives (Powers J. C. et al., Biochem., 24, 2048-2058, (1985)) and peptidic boron derivatives (Kato Y. et al., J. Biochem., 103, 820-822, (1988)). Non-peptidic synthetic inhibitors include diisopropyl fluorophosphate (DFP), 1-1-tosylamino-2-phenyle thylchloromethylketone (TPCK) and α-toluensulfonylfluoride (PMSF), which are commercially available protease inhibitors. However, none of these substances are satisfactory with respect to their inhibitory activity and specificity.

On the other hand, nitric oxide which is generated in an organism works as a physiologically active substance in the circularoty, nervous and immune systems (Nathan C. FASEB J.,6, 3051-3064, (1992)), and it is also noted in the field of inflammation, allergic reaction, diabetes, etc. (Kolb H. et al., Immunology Today, 13, 157-159, (1992); Corbett J. A. et al., J. Clin. Invest., 90, 2384-2391, (1992)).

However, when nitric oxide is generated excessively and released in an organism, it has been reported that various damages on cells and tissues are caused because nitric oxide per se has high chemical reactivity (Kilbourn R. G. et al., J. Natl. Cancer Inst. 84, 827-831, (1992); Nava E. et al., Lancet, 338, 1555-1557 (1991); Thiemermann C. et al., Proc. Natl. Acad. Sci. USA., 90, 267-271, (1993)).

As mentioned above, it is expected that a compound which inhibits nitric oxide production has a potent use as an agent for the prevention and treatment of cardiopathy and cerebrovascular diseases, ishemic heart disease, septic shock, ache, rheumatic fever, arthritis, asthma, immunodeficiency, viral or non-viral infections, autoimmune diseases and cancer.

Accordingly, an object of the present invention is to provide a substance which potently inhibits the activity of a chymotrypsin-like enzyme, more specifically the activity of chymase, and nitric oxide production.

The present invention provides the use of a compound of the following general formula (1) to achieve the inhibition of chymase activity and nitric oxide production.

General formula (1)

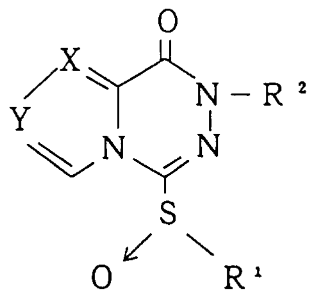

or

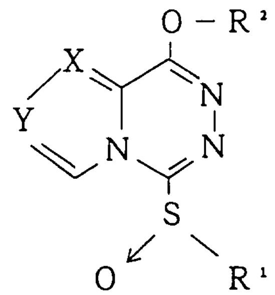

(1a) (1b)

where in formula (1a) X is C-$CH_3$ and Y is N, $R^1$ is a lower alkyl group or a benzyl group substituted by one halogen atom and $R^2$ is a lower alkyl group, a benzyl group substituted by one halogen atom or a lower alkoxycarbonylmethyl group, and where in formula (1b) X is N and Y is CH, X is CH and Y is CH, or X is C-$CH_3$ and Y is N, $R^1$ is a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group, and $R^2$ is a hydrogen atom, a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group.

Groups represented in the above formula (1) are more specifically as follows.

A "halogen atom" includes, for example, chlorine atom and fluorine atom.

An "alkoxycarbonylmethyl group" includes those of which alkoxy has 1 to 6 carbon atoms, for example, ethoxycarbonylmethyl group.

"Lower" denotes from one to six carbon atoms unless otherwise described.

A "lower alkyl group" includes, for example, linear or branched alkyl groups having from one to six carbon atoms such as methyl, ethyl, propyl, butyl, pentyl, hexyl, isopropyl and tert-butyl.

A "halo lower alkyl group" includes, for example, trifluoromethyl group.

A "benzyl group whose phenyl ring is substituted by any one of lower alkyl group, halogen atom, cyano group, phenyl group and halo lower alkyl group" includes 4-methylbenzyl, 4-isopropylbenzyl, 4-tert-butylbenzyl, 4-chlorobenzyl, 3-chlorobenzyl, 4-fluorobenzyl, 4-trifluoromethylbenzyl, 4-cyanobenzyl and 4-phenylbenzyl.

The compound of the present invention can be produced by the following processes.

Reaction formula-1

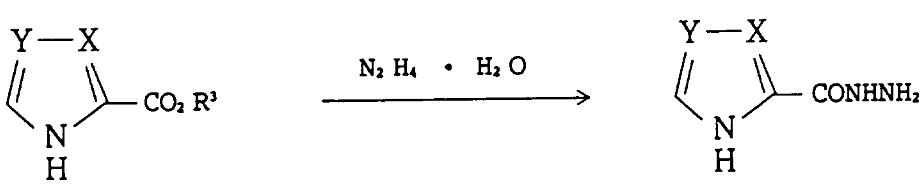


(3) (4)

wherein X and Y are as defined above and $R^3$ is lower alkyl groups such as methyl and ethyl.

The reaction of an alkoxycarbonyl compound (3) and hydrazine hydrate can be conducted in a suitable solvent.

Examples of solvents used in the reaction include lower alcohols such as methanol, ethanol, n-propanol, isopropanol, n-butanol and tert-butanol. The molar ratio of hydrazine hydrate to compound (3) used in the reaction is not restricted to a specific one but may be selected suitably from broad range, for example, at least 5, and preferably from 5 to 10. The reaction is normally conducted at a temperature of from 20 to 150 °C, and preferably from 20 to 100 °C, for one to five hours.

Reaction formula-2

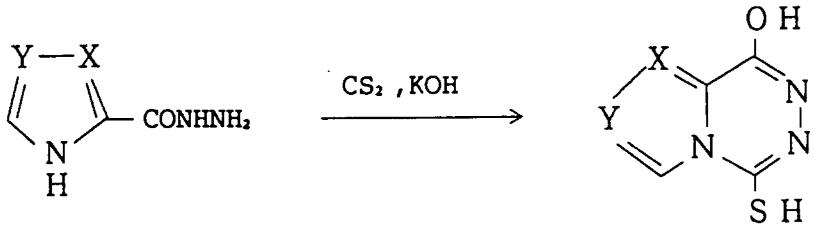


(4) (5)

wherein X and Y are as defined above.

The reaction of a hydrazinocarbonyl compound (4) and carbon disulfide may be conducted in a suitable solvent in the presence or absence of a basic compound.

Examples of solvents used in the reaction include lower alcohols such as methanol, ethanol, n-propanol, isopropanol, n-butanol and tert-butanol, and N,N-dimethylformamide. Examples of the basic compounds include carbonates such as sodium carbonate, potassium carbonate, sodium bicarbonate and potassium bicarbonate, metal hydroxides such as sodium hydroxide and potassium hydroxide, sodium hydride, potassium, sodium, sodium amide, metal alcoholates such as sodium ethylate and sodium methylate, and organic bases such as pyridine, N-ethyldiisopropylamine, 4-dimethylaminopyridine, triethylamine, 1,5-diazabicyclo[4.3.0]non-5-ene (DBN), 1,8-diazabicyclo[5.4.0] undec-7-ene (DBU) and 1,4-diazabicyclo[2.2.2]octane (DABCO). The molar ratio of carbon disulfide to compound (4) used in the reaction is not restricted to a specific one but may be selected suitably from a broad range, for example, at least 2, and preferably from 2 to 10. The reaction is normally conducted at a temperature of from 20 to 150°C , and preferably from 40 to 100 °C, for 10 to 30 hours.

The following reaction formula shows introduction of $R^1$ group, provided that $R^2$ is hydrogen.

Reaction formula-3

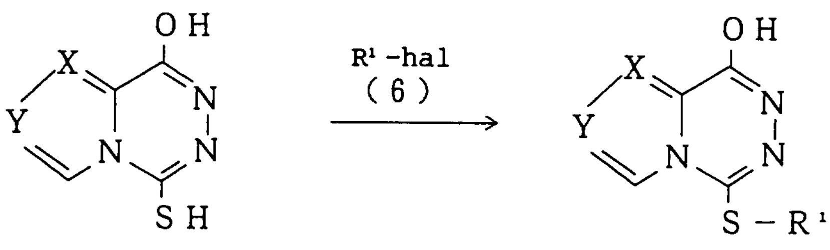


(5) (2b)

wherein $R^1$ , X and Y are as defined above, and "hal" represents halogen (Cl, Br or I).

The reaction of compound (5) and compound (6) may be conducted in a suitable inert solvent in the presence of a basic compound.

Examples of the inert solvents used in the reaction include aromatic hydrocarbons such as benzene, toluene and xylene, ethers such as tetrahydrofuran, dioxane, diethylene glycol dimethylether, lower alcohols such as methanol, ethanol, n-propanol, isopropanol, n-butanol and tert-butanol, ethyl acetate, acetone, acetonitrile, pyridine, dimethylsulfoxide, N,N-dimethylformamide and mixtures thereof. Examples of the basic compounds include carbonates such as sodium carbonate, potassium carbonate, sodium bicarbonate and potassium bicarbonate, metal hydroxides such as sodium hydroxide and potassium hydroxide, sodium hydride, potassium, sodium, sodium amide, metal alcoholates such as sodium ethylate and sodium methylate, and organic bases such as pyridine, N-ethyldiisopropylamine, dimethylaminopyridine, triethylamine, 1,5-diazabicyclo[4.3.0]non-5-ene (DBN), 1,8-diazabicyclo[5.4.0] undec-7-ene (DBU) and 1,4-diazabicyclo[2.2.2]octane (DABCO). The molar ratio of compound (6) to compound (5) used in the reaction is not restricted to a specific one but may be selected suitably from broad range, for example, 1.0, and preferably 1.0 to 1.2.

The reaction is normally conducted at a temperature of from 0 to 50 °C , and preferably from 0 to 20 °C , for 5 to 12 hours.

Compounds synthesized according to the reaction formula-3 are summarized in the following Table 1.

Table 1

| General Formula | Example | $R^1$ |
|---|---|---|
| 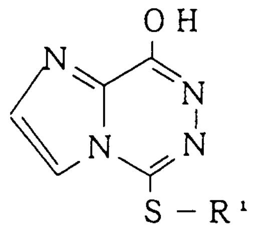 | 1 | 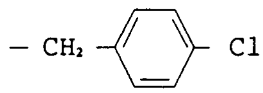 |
| | 2 | 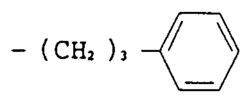 |
| 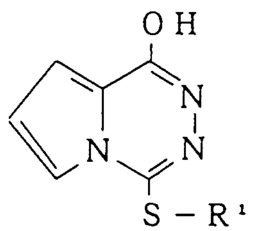 | 3 | 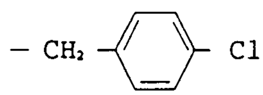 |
| | 4 | 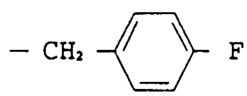 |
| 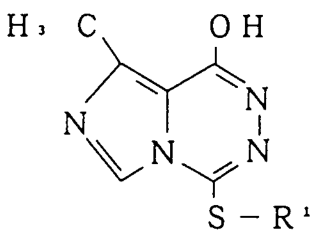 | 5 | 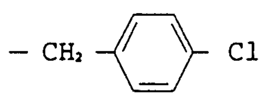 |
| | 6 | 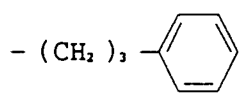 |

The following reaction formula shows the introduction of the $R^2$ group, provided that $R^1$ is different from $R^2$.

Reaction formula-4

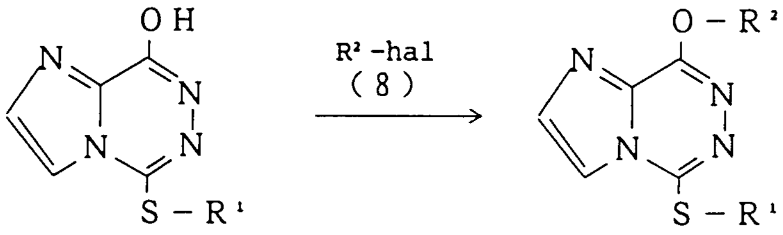


( 7 a )                ( 2 b )

wherein $R^1$ and $R^2$ are as defined above.

The reaction of compound (7a) and compound (8) may be conducted under the same conditions as employed in the reaction of compound (5) and compound (6) according to reaction formula-3.

A compound synthesized according to the reaction formula-4 is shown in the following Table 2.

Table 2

| General Formula | Example | $R^1$ | $R^2$ |
|---|---|---|---|
| 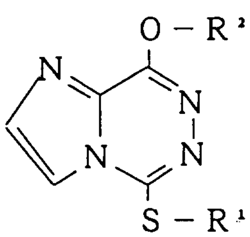 | 27 | 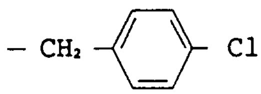 | $-CH_2CO_2C_2H_5$ |

Reaction formula-5

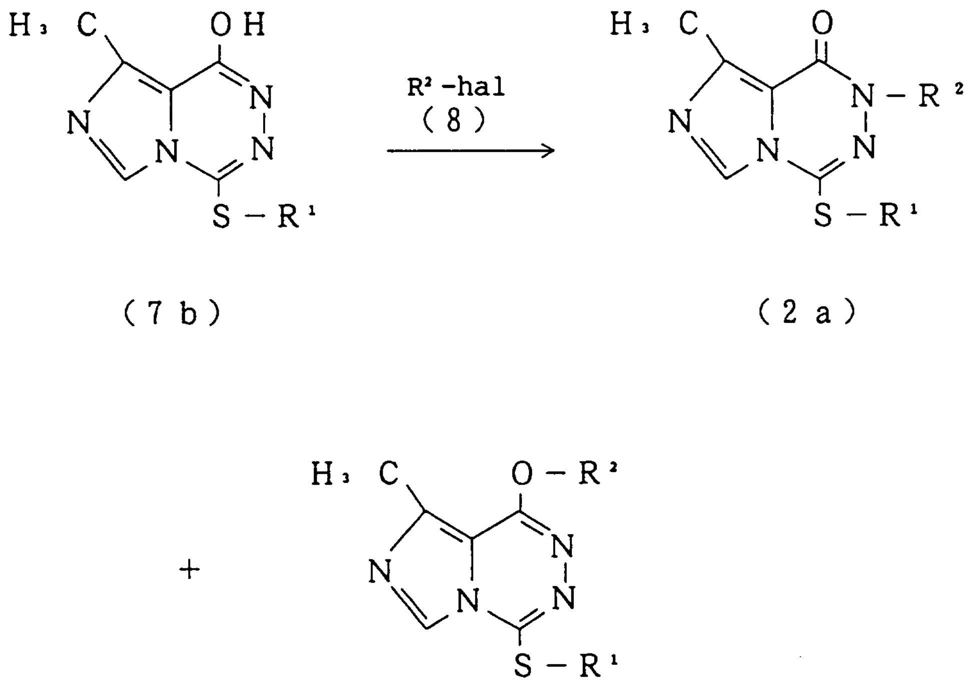


(7b) (2a)

(2b)

wherein $R^1$ and $R^2$ are as defined above.

The reaction of compound (7b) and compound (8) may be conducted under the same conditions as employed in the reaction of compound (5) and compound (6) according to reaction formula-3.

Compounds synthesized according to the reaction formula-5 are summarized in the following Table 3.

Table 3

| General Formula | Example | $R^1$ | $R^2$ |
|---|---|---|---|
| 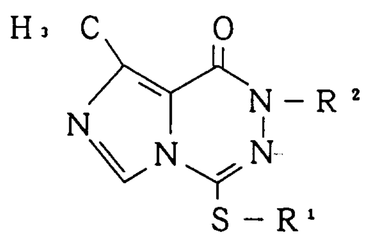 | 28a | 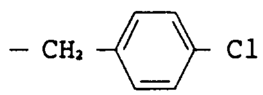 | $-CH_2CO_2C_2H_5$ |
| 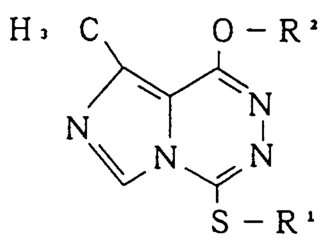 | 28b | 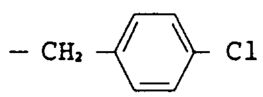 | $-CH_2CO_2C_2H_5$ |

The following reaction formula shows the Introduction of the $R^1$ group, provided that $R^1$ is identical with $R^2$.

Reaction formula-6

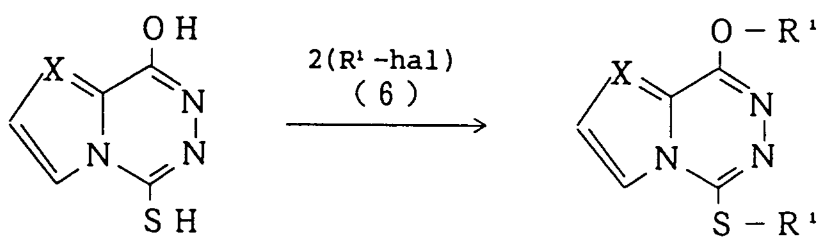


(5a) (2b)

wherein $R^1$ and X are as defined above.

The reaction of compound (5a) and compound (6) may be conducted in a suitable inert solvent in the presence of a basic compound. In this reaction, any solvent and basic compound which are used in the reaction of compound (5) and compound (6) according to the reaction formula-3 may be used. The molar ratio of compound (6) to compound (5a) used in the reaction is normally from 2 to 5, and preferably from 2 to 3. The reaction is conducted normally at a temperature of from 0 to 20 °C, and preferably from 0 to 10 °C, for 5 to 12 hours.

Compounds synthesized according to the reaction formula-6 are summarized in the following Table 4.

Table 4

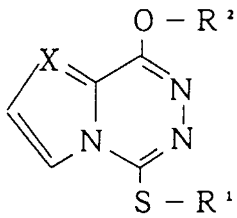

$R^1 = R^2$

General Formula

| Example | X | $R^1$ | Example | X | $R^1$ |
|---|---|---|---|---|---|
| 7 | N | 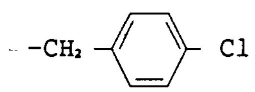 | 16 | N | 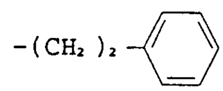 |
| 8 | C | 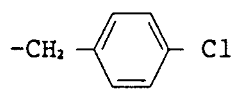 | 17 | N | 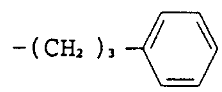 |
| 9 | N | 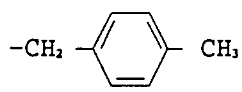 | 18 | N | $-CH_2CH_3$ |
| 10 | N | 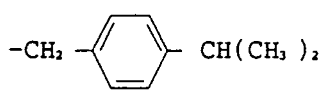 | 19 | N | $-(CH_2)_2CH_3$ |
| 11 | N | 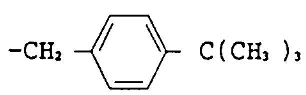 | 20 | N | $-(CH_2)_3CH_3$ |
| 12 | N | 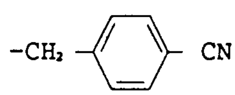 | 21 | N | $-(CH_2)_4CH_3$ |
| 13 | N | 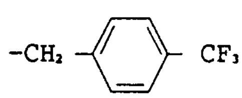 | 22 | N | $-(CH_2)_5CH_3$ |
| 14 | N | 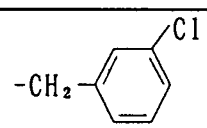 | 23 | N | $-(CH_2)_2CH_2Cl$ |
| 15 | N | 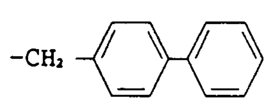 | 24 | N | $-CH_2CO_2C_2H_5$ |

Reaction formula-7

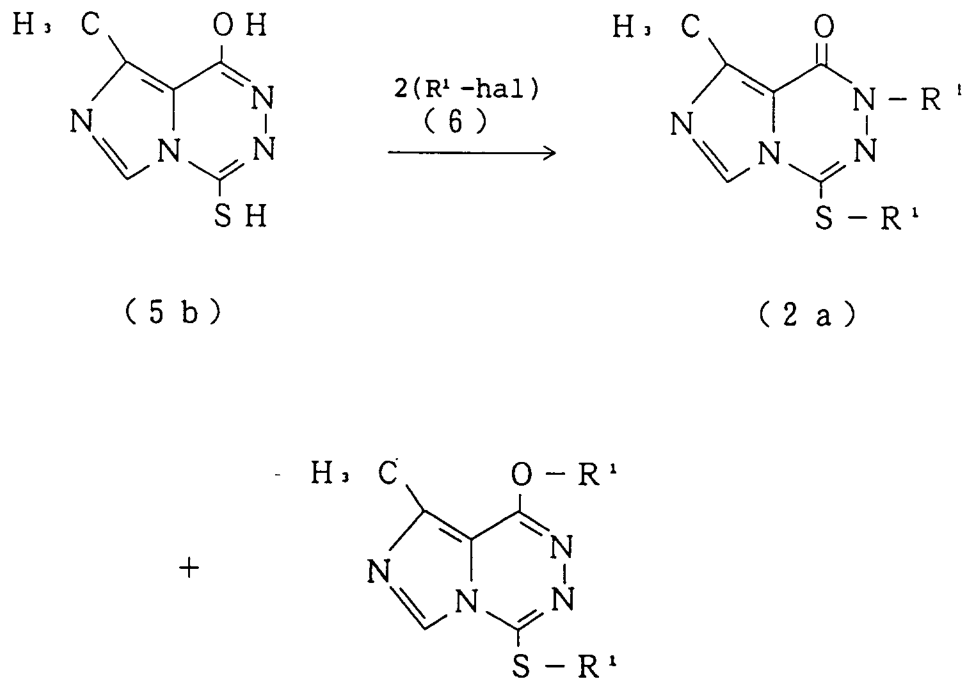


(5b) (2a)

+

(2b)

wherein $R^1$ is as defined above.

The reaction of compound (5b) and compound (6) may be conducted under the same conditions as employed in the reaction of compound (5a) and compound (6) according to reaction formula-6.

Compounds synthesized according to the reaction formula-7 are summarized in the following Table 5.

Table 5

| General Formula | Example | $R^1 = R^2$ |
|---|---|---|
| 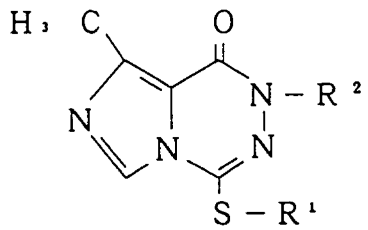 | 25a | 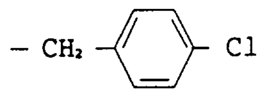 |
| | 26a | $-CH_2CH_3$ |
| 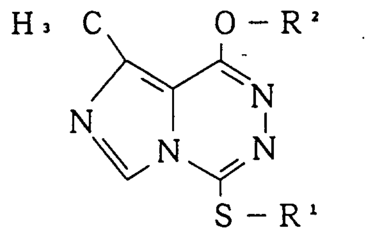 | 25b | 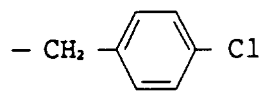 |
| | 26b | $-CH_2CH_3$ |

Reaction formula-8

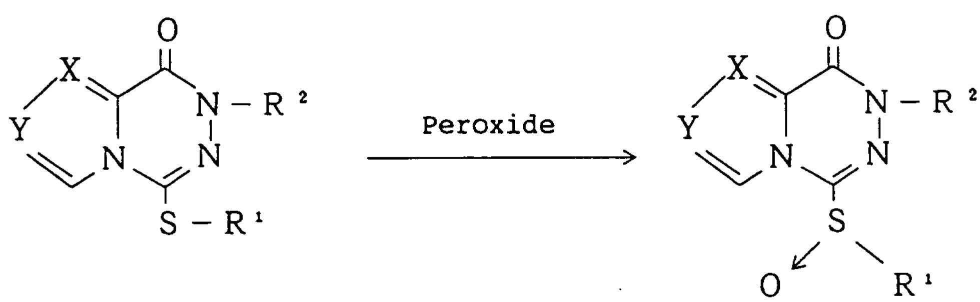


(2a) (1a)

wherein $R^1$, $R^2$, X and Y are as defined above.

The reaction of compound (2a) and a peroxide may be conducted in a suitable solvent. Examples of peroxides used in the reaction include perbenzoic acid, m-chloro-perbenzoic acid, peracetic acid and hydrogen peroxide. Examples of solvents used in the reaction include chloroform and dichloromethane. The molar ratio of peroxide to compound (2a) used in the reaction is not restricted to a specific one but may be selected suitably from broad range, for example, 1.0, and preferably 1.0 to 1.5. The reactrion is normally conducted at a temperature of from 0 to 50 °C , and preferably from 0 to 20 °C, for 2 to 4 hours.

Compounds synthesized according to the reaction formula-8 are summarized in the following Table 6.

Table 6

| General Formula | Example | $R^1$ | $R^2$ |
|---|---|---|---|
| 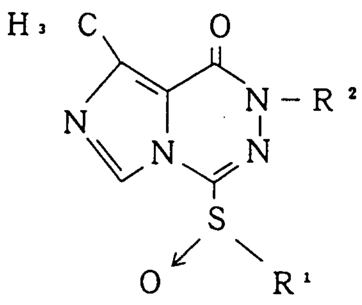 | 54 | $-CH_2$ 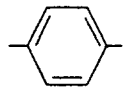 Cl | $-CH_2$ 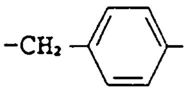 Cl |
| | 56 | $-CH_2CH_3$ | $-CH_2CH_3$ |
| | 59 | $-CH_2$ 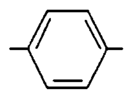 Cl | $-CH_2CO_2C_2H_5$ |

Reaction formula-9

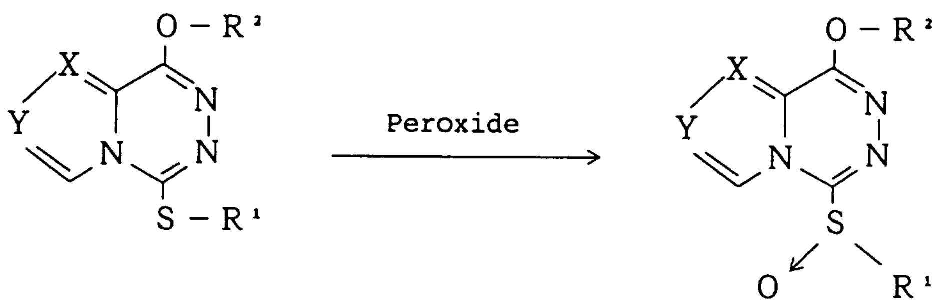


(2b) (1b)

wherein $R^1$, $R^2$, X and Y are as defined above.

The reaction of compound (2b) and peroxide may be conducted under the same conditions as employed in the reaction of compound (2a) and peroxide according to the reaction formula-8.

Compounds synthesized according to the reaction formula-9 are summarized in the following Tables 7 and 8.

Table 7

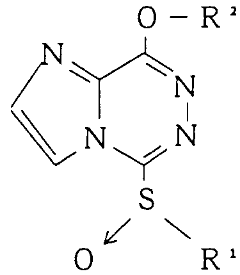

General Formula

| Example | $R^1$ | $R^2$ |
|---|---|---|
| 31 | 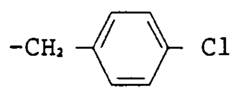 | H |
| 32 | 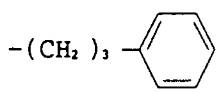 | H |
| 35 | 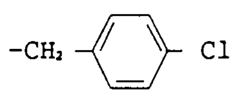 | 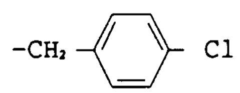 |
| 37 | 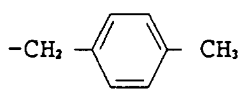 | 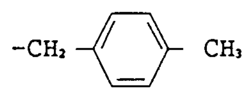 |
| 38 | 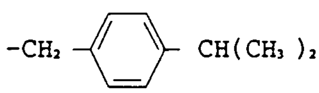 | 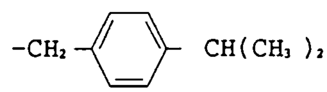 |
| 39 | 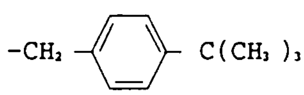 | 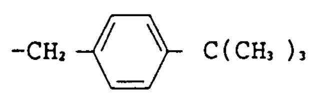 |
| 40 | 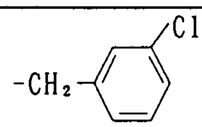 | 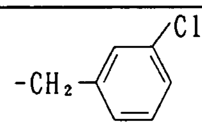 |
| 41 | 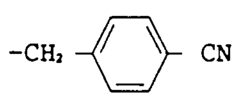 | 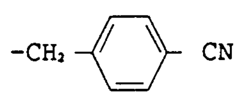 |

Table 7 (Continued)

| Example | $R^1$ | $R^2$ |
| --- | --- | --- |
| 42 | 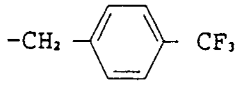 | 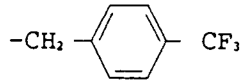 |
| 43 | 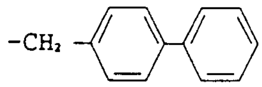 | 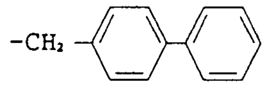 |
| 44 | 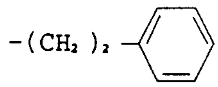 | 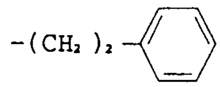 |
| 45 | 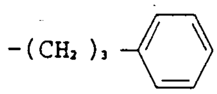 | 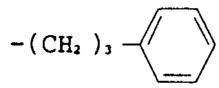 |
| 46 | $-CH_2CH_3$ | $-CH_2CH_3$ |
| 47 | $-(CH_2)_2CH_3$ | $-(CH_2)_2CH_3$ |
| 48 | $-(CH_2)_3CH_3$ | $-(CH_2)_3CH_3$ |
| 49 | $-(CH_2)_4CH_3$ | $-(CH_2)_4CH_3$ |
| 50 | $-(CH_2)_5CH_3$ | $-(CH_2)_5CH_3$ |
| 51 | $-(CH_2)_2CH_2Cl$ | $-(CH_2)_2CH_2Cl$ |
| 52 | $-CH_2CO_2C_2H_5$ | $-CH_2CO_2C_2H_5$ |
| 57 | 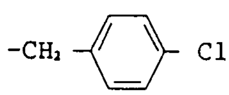 | $-CH_2CO_2C_2H_5$ |

Table 8

| General Formula | Example | $R^1$ | $R^2$ |
|---|---|---|---|
| 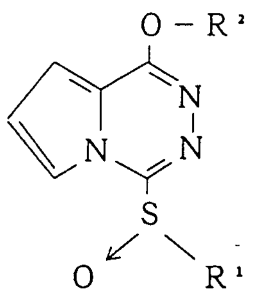 | 29 | $-CH_2$ 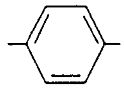 Cl | H |
| | 30 | $-CH_2$ 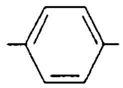 F | H |
| | 36 | $-CH_2$ 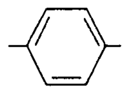 Cl | $-CH_2$ 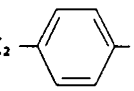 Cl |
| 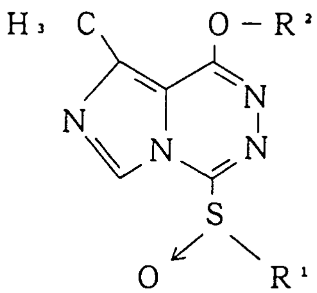 | 33 | $-CH_2$ 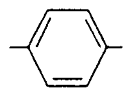 Cl | H |
| | 34 | $-(CH_2)_3$ 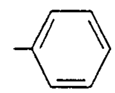 | H |
| | 53 | $-CH_2$ 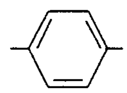 Cl | $-CH_2$ 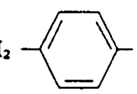 Cl |
| | 55 | $-CH_2CH_3$ | $-CH_2CH_3$ |
| | 58 | $-CH_2$ 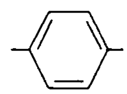 Cl | $-CH_2CO_2C_2H_5$ |

Effects of the Present Invention

The compounds of the present invention have potent inhibitory activities to chymase activity and nitric oxide production, and therefore are useful for the treatment of various disorders, which are caused by elevation of chymase activity or excessive nitric oxide production.

The present invention will further be explained in detail by examples below.

Test Example 1: Measurement of Inhibition of Chymase Activity

[Rat Peritoneal Cavity Mast Cell Preparation]

Mast cells were obtained from the peritoneal cavity of male Sprague Dawley(SD) rats by the method of Graziano et al. (Methods in Enzymol., 162, 501-514, 1988), layered on 75% Percoll (Pharmacia Biotech), and then purified to over 90% purity by centrifugation at 150xG for 15 min.

[Chymase Purification]

Chymase was partially purified from rat peritoneal cavity mast cells by the method of Kido et al. (Arch. Biochem. Biophys., 239, 436-443, 1985). Purified mast cells were quickly frozen and thawed several times and homogenized in a low ionic strength-buffer (pH6.5). The homogenate was centrifuged at 20,000xG for 20 min. The precipitate was re-homogenized in a high ionic strength-buffer (pH8.0) and cetrifuged at 20,000xG for 20 min. The supernatant was recovered and the enzyme was extracted. From the crude extract, chymase was partially purified by an Octyl-Sepharose CL-4B (Pharmacia Biotech) hydrophobic chromatography.

[Assay of Inhibition of Chymase Activity]

Ten microliters of test compounds in dimethyl sulfoxide (DMSO) were mixed with 250 µl of 60 mM Tris-HCl buffer (pH8.0) containing 0.2 M NaCl and 250 µl of 1 µg/ml chymase in buffer. The mixtures were pre-incubated for 5 min. at 37°C . After the addition of 500 µl of 0.6 mM chromogenic substrate, N-Suc-Ala-Ala-Pro-Phe-p-nitroanilide (Sigma), the reactions were carried out for 30 min. at 37 °C , and then stopped by the addition of 50 µl of 0.75 M acetic acid. The absorbances at 405nm were measured in a spectrophotometer, and the amount of p-nitroaniline released from the substrate was measured.

Calculations of the inhibition% of chymase by test compounds were carried out as below.

$$\text{Inhibition\%} = \{1-(a-a')/(b-b')\} \times 100\ (\%)$$

a ; the absorbance in presence of test compound.
a' ; the absorbance of non-reaction in presence of test compound.
b ; the absorbance in absence of test compound.
b' ; the absorbance of non-reaction in absence of test compound.

$IC_{50}$ values were expressed as the concentrations (M) of test compounds producing 50% inhibition.

Table 9 shows the results of chymase inhibition by compounds of the present invention.

Table 9

| Test Compound (Example No.) | Inhibition of Chymase $IC_{50}$ (M) | Test Compound ( Example No.) | Inhibition of Chymase $IC_{50}$ (M) |
|---|---|---|---|
| 29 | $3 \times 10^{-7}$ | 45 | $9 \times 10^{-7}$ |
| 30 | $2 \times 10^{-6}$ | 46 | 5 % * |
| 31 | $5 \times 10^{-8}$ | 47 | $7 \times 10^{-6}$ |
| 32 | $2 \times 10^{-7}$ | 48 | $3 \times 10^{-6}$ |
| 33 | $6 \times 10^{-7}$ | 49 | $2 \times 10^{-7}$ |
| 34 | $8 \times 10^{-6}$ | 50 | $3 \times 10^{-6}$ |
| 35 | $3 \times 10^{-8}$ | 51 | 50% * |
| 36 | $3 \times 10^{-7}$ | 52 | 39% * |
| 37 | $9 \times 10^{-8}$ | 53 | $6 \times 10^{-8}$ |
| 38 | 41% * | 54 | $5 \times 10^{-8}$ |
| 39 | - 3% * | 55 | 22% * |
| 40 | $4 \times 10^{-8}$ | 56 | 8% * |
| 41 | $2 \times 10^{-6}$ | 57 | $5 \times 10^{-8}$ |
| 42 | 11%* | 58 | $8 \times 10^{-8}$ |
| 43 | $5 \times 10^{-6}$ | 59 | $3 \times 10^{-8}$ |
| 44 | $6 \times 10^{-7}$ | | |

* Inhibition (%) of chymase at a concentration of $10^{-5}$ M

The data shown in Table 9 clearly indicate that the compounds of the present invention exhibit potent inhibitory activity against rat mast cell chymase.

Test Example 2: Inhibition of Histamine Release

[Preparation of Antiserum]

Male SD rats (7-week-old) were pre-housed and their spleens were excised. After 3 days, the rats were immunized by injecting 0.25 ml of 1 mg/ml ovalbumin (OA, Seikagaku Kogyo) with killed organisms of Bordetella pertussis (Kaken Pharm.) into the 4 parts of foot pad subcutaneously. After 14 days, blood was collected and centrifuged to separate the anti-OA serum. The anti-OA IgE antibody titer of the antiserum was 1:160 as estimated by 72-h homologous passive cutaneous anaphylaxis (PCA) in rats challenged with OA.

[Assay of Histamine Release Inhibition]

Purified peritoneal cavity rat mast cells ($1x10^7$ cells) were suspended in 15 ml of anti-OA serum and incubated for 60 min at 37 °C . Then the cells were washed three times with phosphate buffered saline containing 0.1% gelatin (PBS-G, pH7.4) followed by centrifugation and suspended at a concentration of $1x10^5$ cells/ml in PBS-G. Nine hundreds microliters of mast cell suspensions were mixed with 1 µl of test compounds in dimethylsulfoxide and pre-incubated in a final volume of 1.0 ml for 30 min. Then 100 µg/ml of OA were added to induce release of histamine. After 20 min.,the reactions were stopped by cooling in ice-cold bath. The mixtures were centrifuged at 100xG for 10 min at 4°C . The supernatant fractions were used for fluorometric measurement of histamine by the modified method of Shore et al.(J. Pharmacol. Exp. Ther., 127, 182-186, 1959). One point five milliliters of 0.01 N HCl were added to 0.4 ml of the supernatants, followed by 0.4 ml of 1 N NaOH and 0.1 ml of 1% o-phthalaldehyde in methanol. The solutions were stood in ice-cold bath for 60 min. After the additions of 0.3 ml of 2 N citric acid, the fluorescences at 450 nm resulting from

activations at 350 nm were measured in a spectrofluorometer. Calculations of the inhibition% of hitamine release by the test compounds were carried out as below.

$$Inhibition\% = \{1-(a-a')/(b-b')\} \times 100(\%)$$

a ; the fluoresence in presence of test compound
a' ; the fluoresence of reaction without OA in presence of test compound
b ; the fluoresence in absence of test compound
b' ; the fluoresence of reaction without OA in presence of test compound

$IC_{50}$ values were expressed as the concentrations (M) of test compounds producing 50% inhibition.

Table 10 shows the results of inhibition of histamine release by compounds of the present invention.

Table 10

| Test Compound (Example No.) | Inhibition of Histamine Release $IC_{50}$ (M) |
|---|---|
| 29 | $3 \times 10^{-6}$ |
| 31 | $1 \times 10^{-7}$ |
| 35 | $4 \times 10^{-8}$ |
| Sodium Cromoglicate | $6 \times 10^{-4}$ |

As will be seen in Table 10, compounds of the present invention markedly inhibited IgE-mediated histamine release from rat mast cells.

Test Example 3: Measurement on Inhibition against Nitric Oxide (NO) Production of Macrophage Cell induced by L ipopolysaccharide (LPS)

[Cell Culture and LPS-Stimulation]

The mouse macrophage cell line, RAW264, obtained from the Riken Cell Bank, was cultured in RPMI-1640 medium without phenol red containing 10% inactivated fetal bovine serum and 100 µg/ml kanamycin at 37°C in 5% $CO_2$ in air. Cells were seeded into 96-well tissue culture plates ($6 \times 10^4$ cells/well) and allowed to adhere for 4 hr at 37 °C . To assess the effects of test compounds on induction of NO production, 100 µl of fresh culture medium containing them were added to the medium, followed by the additions of 1 µg/ml of LPS (E Coli 055, derived from B5 cell line, DIFCO Co.) to activate macrophage cells. At the same time, cultivation was conducted in the presence or absence of a test compound.

[Assay of NO Production Inhibition]

Since the measurement of NO is difficult due to the unstableness of NO, the nitrite ion ($NO_2^-$) which is the oxidation product of NO was measured.

Productions of NO were assayed by measuring the concentrations of $NO_2^-$ in culture medium by the Griess reaction by the method of Green et al. (Anal. Biochem., 126, 131-138, 1982). Nitrite accumulation in the cell culture medium was measured 36hr after LPS-stimulations. One hundred microliters of Griess reagents (1% sulfanylamide and 0.1% N-1-naphthylethylenediamine dichloride in 5% phosphoric acid) were added to 100 µl of cell culture medium at a room temperature. After 10 min, the optical densities at 550 nm (OD550) were measured by using a microplate reader.

In unstimulated RAW264 macrophages, $NO_2^-$ production for 36 hr was not determined in the the presence or absence of test compounds. Calculations of the inhibition% of NO production by the test compounds were carried out as depicted below.

$$Inhibition\% = (1-a/b) \times 100(\%)$$

a ; the OD550 in presence of test compound

b ; the OD550 in absence of test compound

$IC_{50}$ values were expressed as the concentrations (M) of test compounds producing 50% inhibition.

Table 11 shows the results of inhibition of NO production by compounds of the present invention.

Table 11

| Test Compound (Example No.) | Inhibition of NO Production $IC_{50}$ (M) |
|---|---|
| 29 | $6 \times 10^{-7}$ |
| 31 | $3 \times 10^{-6}$ |
| 33 | 4 * |
| 35 | 20 * |
| 36 | 31 * |
| 40 | 32 * |
| 41 | 49 * |
| 42 | 8 * |
| 46 | $2 \times 10^{-6}$ |
| 53 | $3 \times 10^{-6}$ |
| 54 | $3 \times 10^{-6}$ |
| 55 | $1 \times 10^{-6}$ |
| 56 | $3 \times 10^{-6}$ |
| 59 | $3 \times 10^{-6}$ |

* Inhibition (%) of NO production at the concentration of $10^{-5}$ M

As will be seen in Table 11, the compounds of the present invetion markedly inhibited LPS-stimulated NO production from mouse macrophages, RAW264.

Test Example 4: Single-Dose Toxicity

Ten Male ICR mice (4-week-old) were housed in each group. Test compounds were suspended in 0.5% aqueous sodium methyl cellulose, and then administered p.o. at the dose of 500 mg/kg to mice. Animals were then observed for 14 days and the mortality rate was determined. Table 12 shows the results of single-dose toxicity of the compounds of the present invention.

Table 12

| Test Compound (Example No.) | The number of death/ The number of animal |
|---|---|
| 29 | 0/10 |
| 31 | 0/10 |
| 35 | 0/10 |
| 53 | 0/10 |
| 54 | 0/10 |

As will be seen in Table 12, no deaths are recorded for any of the mice. Thus, for the compounds of the present invention no acute toxicity was observed.

ADMINISTRATION

For the purpose mentioned above, the compounds of the present invention, described in the general formula (1) may normally be administered systemically or parenterally, usually by oral or parenteral administration.

The dose to be administered is determined depending upon age, body weight, symptom, the desired therapeutic effect, the route of administration, and the duration of the treatment etc. In the human adult, the doses per person for one time are generally between 10 mg and 500 mg, by oral administration up to several times per day, and between 1 mg and 200 mg, by parenteral administration up to several times per day.

As mentioned above, the doses to be used depend on various conditions. Therefore, there are cases in which doses lower than the ranges specified above and doses greater than the ranges specified above, may be used.

The compositions for oral or parenteral administration are produced by conventional methods, using general pharmaceutical carriers. Pharmaceutical carriers employed may either be solid or liquid. Solid carriers include lactose, kaolin, sucrose, talc, gelatin, agar, pectin, acacia, magnesium stearate, stearic acid, while liquid carriers include silop, peanut oil, olive oil and water, as are appropriate to the nature of the active ingredient and the particular form of administration desired. Similarly carriers or diluents include waxing agents such as glyceryl monostearate,glyceryl distearate. Preparation of injection formulations or eye drops may be combined with a pH regulator, buffers, stabilizers, solubilizer, and if necessary these active compounds may be freeze-dryed. These active compounds may be administered sabcutaneously or intramuscularly or intravenously or by instillation.

Examples

The compounds of this invention and their preparation can be understood further by the following examples, but should not constitute a limitation thereof.

Reference example 1

2-hydrazinocarbonylimidazole

2-ethoxycarbonylimidazole was prepared according to the method given in the literature, J. Org. Chem., Vol.43, No.22, 4381 Kenneth L. kirk.

80% hydrazine hydrate (4.6g, 75mmol) was added dropwise to a solution of 2-ethoxycarbonylimidazole (2.1g, 15mmol) in ethanol (20ml) at room temperature and stirring was continued for 2 h. The precipitate was filtered, washed with water and dried to give the title compound (1.7g, 90.0%).

Reference example 2

2-hydrazinocarbonylpyrrole

The title compound was prepared according to the method given in the literature, J. Am. Chem. Soc., Vol.75, 1933, (1953) Harry L. Yall et al.

Reference example 3

5-methyl-4-hydrazinocarbonylimidazole

5-methyl-4-ethoxycarbonylimidazole (30.8g, 0.2mol) was dissolved in 80% hydrazine hydrate (120ml). The reaction mixture was refluxed for 4 h. The reaction mixture was cooled, the precipitate was filtered, washed with water and dried to give the title compound (23.6g, 84.4%).

Reference example 4

8-hydroxy-5-mercaptoimidazo[1.2-d][1.2.4]triazine

To the suspension of 2-hydrazinocarbonylimidazole (3.7g, 30mmol) in ethanol (250ml) carbon disulfide (11.4g, 0.15mol) was added in several portions. The reaction mixture was stirred for 1.5 h. The reaction mixture was evaporated and the residue was dissolved in water (150ml). The reaction mixture was acidified with acetic acid. The precipitate was filtered, washed with water and dried to give the title compound (4.3g, 86.9%).

Reference example 5

1-hydroxy-4-mercaptopyrrolo[1,2-d][1.2.4]triazine

The title compound was prepared in the same manner as described in reference example 4 , by use of 2-hydrazinocarbonylpyrrole in place of 2-hydrazinocarbonylimidazole (64.6%).

Reference example 6

1-hydroxy-4-mercapto-8-methylimidazo[1,5-d][1.2.4]triazine

The title compound was prepared in the same manner as described in reference example 4, by use of 5-methyl-4-hydrazinocarbonylimidazole in place of 2-hydrazinocarbonylimidazole (71.6%).

Example 1

5-(4-chlorobenzylthio)-8-hydroxyimidazo[1,2-d][1.2.4]triazine

To the solution of 8-hydroxy-5-mercaptoimidazo[1,2-d][1.2.4]triazine (0.5g, 3mmol) in N,N-dimethyl formamide (15ml) 95% potassium hydroxide (0.2g, 3.6mmol) and 4-chlorobenzyl chloride (0.53g, 3.4mmol) were added under cooling in an ice-bath. The reaction mixture was stirred for 6 h at room temperature. The reaction mixture was poured into ice-water. The precipitate was filtered, washed with water and dried to give the title compound (0.6g, 77.4%).

| | |
|---|---|
| 1H-NMR($CDCl_3$) δ | 4.49 (2H,s), 7.23 (2H,d,J=4.88Hz), 7.30 (2H,d,J=8.31Hz), 7.41 (2H,d,J=8.31Hz) |
| IRν $_{max}$ $cm^{-1}$ (KBr) | 3400, 1620, 1530, 1485, 1465, 1445, 1215, 1130, 1110, 1090 |
| m.p. | 237.5-239.5°C |

Example 2

8-hydroxy-5-(3-phenylpropylthio)imidazo[1,2-d][1.2.4]triazine

To the solution of 8-hydroxy-5-mercaptoimidazo[1,2-d][1.2.4]triazine (0.3g, 2mmol) in N,N-dimethyl formamide (15ml) anhydrous potassium carbonate (0.2g, 2.1mmol) and 1-bromo-3-phenylpropane (0.4g, 2.1mmol) were added under cooling in an ice-bath. The reaction mixture was poured in to ice-water. The precipitate was filtered, washed with water and dried to give the title compound (0.3g, 44.5%).

| | |
|---|---|
| 1H-NMR($CDCl_3$) δ | 2.18 (2H,quint,J=7.33Hz), 2.80(2H,t,J=7.33Hz), 3.30 (2H,t,J=7.32Hz), 7.18-7.35 (7H,m) |
| IRν $_{max}$ $cm^{-1}$ (KBr) | 2720, 2625, 1620, 1540, 1480, 1450, 1410, 1220, 1140, 1110 |
| m.p. | 158.0-160.0°C |

Example 3

4-(4-chlorobenzylthio)-1-hydroxypyrrolo[1,2-d][1.2.4]triazine

To the solution of 1-hydroxy-4-mercaptopyrrolo[1,2-d][1.2.4]triazine (1.6g, 10mmol) in N,N-dimethyl formamide (30ml) 90% potassium tert-butoxide (1.5g, 12mmol) and 4-chlorobenzyl chloride (1.9g, 12mmol) were added under cooling in an ice-bath. The reaction mixture was stirred for 6 h at room temperature. The reaction mixture was poured into ice-water. The precipitate was filtered, washed with water and dried to give the title compound (2.8g, 97.2%).

| | |
|---|---|
| 1H-NMR($CDCl_3$) δ | 4.43 (2H,s), 6.31-6.34 (1H,m), 6.80-6.86(1H,m), 7.01-7.04 (1H,m), 7.23-7.64 (5H,m), 9.76 (1H,br-s) |
| IRν $_{max}$ $cm^{-1}$ (KBr) | 3170, 3120, 1630, 1610, 1530, 1490, 1410, 1200, 1140, 1100 |
| m.p. | 162.0-163.0°C |

Example 4

4-(4-fluorobenzylthio)-1-hydroxypyrrolo[1,2-d][1.2.4]triazine

The title compound was prepared in the same manner as described in example 1, by use of 1-hydroxy-4-mercaptopyrrolo [1,2-d][1.2.4]triazine in place of 8-hydroxy-5-mercaptoimidazo [1,2-d][1.2.4]triazine and 4-fluorobenzyl chloride in place of 4-chlorobenzyl chloride. Yield: 83.7%.

| | |
|---|---|
| 1H-NMR($CDCl_3$) δ | 4.45 (2H,s), 6.26-6.35 (1H,m), 6.81-7.16 (4H,m), 7.38-7.64 (4H,m), 9.90 (1H,br-s) |
| IRν $_{max}$ $cm^{-1}$ (KBr) | 3430, 3170, 3120, 1620, 1610, 1510, 1485, 1220, 1130, 1070 |
| m.p. | 120.0-122.0°C |

Example 5

4-(4-chlorobenzylthio)-1-hydroxy-8-methylimidazo[1,5-d][1.2.4]triazine

The title compound was prepared in the same manner as described in example 3, by use of 1-hydroxy-4-mercapt-8-methylimidazo[1,5-d][1.2.4]triazine in place of 1-hydroxy-4-mercaptopyrrolo[1,2-d][1.2.4]triazine. Yield: 79.7%.

| | |
|---|---|
| 1H-NMR($CDCl_3$) δ | 2.46 (3H,s), 3.31 (1H,s), 4.51 (2H,s), 7.39 (2H,d,J=8.30Hz), 7.48 (2H,d,J=8.30Hz), 7.72 (1H,s) |
| IRν $_{max}$ $cm^{-1}$ (KBr) | 3400, 3050, 2925, 2850, 1620, 1480, 1405, 1320, 1220, 1095, 1070, 1015, 960 |
| m.p. | 178.0-180.5°C |

Example 6

1-hydroxy-8-methyl-4-(3-phenylpropylthio)imidazol[1,5-d][1.2.4]triazine

The title compound was prepared in the same manner as described in example 2, by use of 1-hydroxy-4-mercapto-8-methylimidazo[1,5-d][1.2.4] triazine in place of 8-hydroxy-5-mercaptoimidazo[1,2-d][1.2.4]triazine. Yield: 45.8%.

| | |
|---|---|
| 1H-NMR($CDCl_3$) δ | 2.16 (2H,quint,J=7.57Hz), 2.64 (3H,s), 2.79 (2H,t,J=7.57Hz), 3.27 (2H,t,J=7.32Hz), 7.17-7.31 (5H,m), 7.65 (1H,s) |
| IRν $_{max}$ $cm^{-1}$ (KBr) | 1610, 1480, 1310, 1250, 1220, 1140, 1050, 950 |
| m.p. | 165.0-167.0°C |

Example 7

8-(4-chlorobenzyloxy)-5-(4-chlorobenzylthio)imidazo [1,2-d][1.2.4]triazine

To a solution of 8-hydroxy-5-mercaptoimidazo[1,2-d][1.2.4] triazine (1.0g, 6mmol) in N,N-dimethyl formamide (30ml) 90% potassium tert-butoxide (2.0g, 15mmol) and 4-chlorobenzyl chloride (2.3g, 14mmol) were added under cooling in an ice-bath. The reaction mixture was stirred for 24h at room temperature. The reaction mixture was poured into ice-water. The precipitate was filtered, washed with water and dried to give the title compound (2.1g, 82.6%).

| | |
|---|---|
| 1H-NMR($CDCl_3$) δ | 4.47 (2H,s), 5.71 (2H,s), 7.10-7.41 (10H,m) |
| IRν $_{max}$ $cm^{-1}$ (KBr) | 3430, 1600, 1490, 1465, 1425, 1280, 1215, 1165, 1090, 1060, 1010 |
| m.p. | 152.5-154.0°C |

Example 8

8-(4-chlorobenzyloxy)-4-(4-chlorobenzylthio)pyrrolo[1,2-d][1.2.4]triazine

The title compound was prepared in the same manner as described in example 7, by use of 1-hydroxy-4-me rcaptopyrrolo[1,2-d][1.2.4]triazine in place of 8-hydroxy-5-mercapto imidazo[1,2-d][1.2.4] triazine. Yield: 78.5%.

| | |
|---|---|
| 1H-NMR($CDCl_3$) δ | 4.41 (2H,s), 5.62 (2H,s), 6.28 (1H,dd,J=2.57Hz), 6.83 (1H,dd,J=1.84Hz), 6.88-6.90 (1H,m), 7.05 (2H,d,J=8.43Hz), 7.24-7.31 (3H,m), 7.36 (2H,d,J=8.43Hz) |
| IRν $_{max}$ $cm^{-1}$ (KBr) | 1600, 1490, 1470, 1400, 1190, 1070, 1010 |

| | |
|---|---|
| m.p. | 130.5-132.0°C |

Example 9

1-(4-methylbenzyloxy)-4-(4-methylbenzylthio)imidazo[1,2-d][1.2.4]triazine

The title compound was prepared in the same manner as described in example 7, by use of α-chloro-4-xylene in place of 4-chlorobenzyl chloride. Yield: 79.4%.

| | |
|---|---|
| 1H-NMR($CDCl_3$) δ | 2.32 (6H,s), 4.49 (2H,s), 5.69 (2H,s), 7.06 (1H,s), 7.12-7.25 (7H,m), 7.32 (2H, d, J=8.06Hz) |
| IRν $_{max}$ $cm^{-1}$ (KBr) | 3100, 2900, 1600, 1520, 1480, 1460, 1420, 1210, 1160, 1100, 1060 |
| m.p. | 149.0-151.0°C |

Example 10

8-(4-isopropylbenzyloxy)-5-(4-isopropylbenzylthio)imidazo [1,2-d][1.2.4]triazine

The title compound was prepared in the same manner as described in example 7, by use of 4-isopropylbenzyl chloride in place of 4-chlorobenzyl chloride. Yield: 18.5%.

| | |
|---|---|
| 1H-NMR($CDCl_3$) δ | 1.21 (3H,s), 1.22 (3H,s), 1.24 (3H,s), 1.25 (3H,s), 2.88 (2H,quint,J=6.84Hz), 4.51 (2H,s), 5.70 (2H,s), 7.18-7.25(7H,m), 7.37 (2H,d,J=8.06Hz) |
| IRν $_{max}$ $cm^{-1}$ (KBr) | 2950, 1600, 1480, 1420, 1270, 1210, 1140, 1100, 1080, 1000 |
| m.p. | 97.0-101.0 °C |

Example 11

8-(4-tert-butylbenzyloxy)-5-(4-tert-butylbenzylthio)imidazo [1,2-d][1.2.4]triazine

The title compound was prepared in the same manner as described in example 7, by use of 4-tert-butylbenzyl chloride in place of 4-chlorobenzyl chloride. Yield: 80.6%.

| | |
|---|---|
| 1H-NMR($CDCl_3$) δ | 1.29 (9H,s), 1.30 (9H,s), 4.51 (2H,s), 5.71 (2H,s), 7.09 (1H,s), 7.17-7.24 (3H,m), 7.34-7.40 (6H,m) |
| IRν $_{max}$ $cm^{-1}$ (KBr) | 2950, 1600, 1520, 1480, 1420, 1280, 1200, 1100, 1060 |
| m.p. | 154.0-156.0°C |

Example 12

8-(4-cyanobenzyloxy)-5-(4-cyanobenzylthio)imidazo [1,2-d][1.2.4]triazine

The title compound was prepared in the same manner as described in example 7, by use of α-bromo-4-tolunitrile in place of 4-chlorobenzyl chloride. Yield: 75.0%.

| | |
|---|---|
| 1H-NMR($CDCl_3$) δ | 4.52 (2H,s), 5.81 (2H,s), 7.15 (1H,s), 7.23-7.34 (3H,m) , 7.56-7.65(6H,m) |
| IRν $_{max}$ $cm^{-1}$ (KBr) | 3350, 2210, 1610, 1470, 1420, 1270, 1170, 1100 |
| m.p. | 189.0-191.0°C |

Example 13

8-(4-trifluoromethylbenzyloxy)-5-(4-trifluoromethylbenzylthio) imidazo[1,2-d][1.2.4]triazine

The title compound was prepared in the same manner as described in example 7, by use of α'-bromo-α,α,α-trifluoro-4-xylene in place of 4-chlorobenzyl chloride. Yield: 75.0%.

| | |
|---|---|
| 1H-NMR($CDCl_3$) δ | 4.54 (2H,s), 5.82 (2H,s), 7.13 (1H,s), 7.32 (3H,d,J=7.57Hz), 7.56-7.65 (6H,m) |
| IRν $_{max}$ $cm^{-1}$ (KBr) | 3450, 1600, 1470, 1330, 1170, 1140, 1070 |
| m.p. | 114.0-115.0°C |

Example 14

8-(3-chlorobenzyloxy)-5-(3-chlorobenzylthio)imidazo[1,2-d][1.2.4]triazine

The title compound was prepared in the same manner as described in example 7, by use of 3-chlorobenzyl chloride in place of 4-chlorobenzyl chloride. Yield: 58.0%.

| | |
|---|---|
| 1H-NMR($CDCl_3$) δ | 4.48 (2H,s), 5.73 (2H,s), 7.11(2H,s), 7.20 (1H,s), 7.45 (1H,s), 7.54-7.64 (6H,m) |
| IRν $_{max}$ $cm^{-1}$ (KBr) | 3450, 1700, 1470, 1420, 1270, 1210, 1090 |
| m.p. | 105.0-106.0°C |

Example 15

8-(4-phenylbenzyloxy)-5-(4-phenylbenzylthio)imidazo[1,2-d][1.2.4]triazine

The title compound was prepared in the same manner as described in example 7, by use of 4-(chloromethyl)biphenyl in place of 4-chlorobenzyl chloride. Yield: 60.9%.

| | |
|---|---|
| 1H-NMR($CDCl_3$) δ | 4.58 (2H,s), 5.79 (2H,s), 7.14 (1H,d,J=1.25Hz), 7.28-7.85 (19H,m) |
| IRν $_{max}$ $cm^{-1}$ (KBr) | 3025, 1600, 1485, 1470, 1410, 1260, 1090, 1055, 1000 |
| m.p. | 109.0-111.0°C |

Example 16

8-phenethyloxy-5-phenethylthioimidazo[1,2-d][1.2.4]triazine

The title compound was prepared in the same manner as described in example 7, by use of 2-chloroethylbenzene in place of 4-chlorobenzyl chloride. Yield: 69.1%.

| | |
|---|---|
| 1H-NMR($CDCl_3$) δ | 3.15 (4H,q,J=7.32Hz), 3.55 (2H,t,J=7.32Hz), 4.74 (2H,t,J=7.32Hz), 6.88 (1H,d,J=1.20Hz), 7.11-7.36 (11H,m) |
| IRν $_{max}$ $cm^{-1}$ (NaCl) | 3025, 2925, 1600, 1495, 1465, 1450, 1435, 1260, 1195, 1140, 1090, 1060 |

Example 17

8-(3-phenylpropoxy)-5-(3-phenylpropylthio)imidazo[1,2-d][1.2.4]triazine

The title compound was prepared in the same manner as described in example 7, by use of 1-bromo-3-phenylpropane in place of 4-chlorobenzyl chloride. Yield: 96.0%.

| | |
|---|---|
| 1H-NMR($CDCl_3$) δ | 2.13-2.25 (4H,m), 2.69 (2H,t,J=7.32Hz), 2.80 (2H,t,J=7.33Hz), 3.30 (2H,t,J=7.32Hz), 4.53 (2H,t,J=7.32Hz), 7.14-7.35(12H,m) |
| IRν $_{max}$ $cm^{-1}$ (KBr) | 3025, 2950, 1610, 1480, 1280, 1200, 1100, 920 |
| m.p. | 64.0-65.0°C |

Example 18

8-ethoxy-5-ethylthioimidazo[1,2-d][1.2.4]triazine

The title compound was prepared in the same manner as described in example 7, by use of 1-bromo ethane in place of 4-chlorobenzyl chloride. Yield: 52.0%.

| | |
|---|---|
| 1H-NMR($CDCl_3$) δ | 1.49 (3H,t,J=7.32Hz), 1.51 (3H,t,J=7.32Hz), 3.32 (2H,q,J=7.32Hz), 4.56 (2H,q,J=7.32Hz), 7.14 (1H,s), 7.24(1H,s) |
| IRν $_{max}$ $cm^{-1}$ (KBr) | 3100, 2950, 1600, 1470, 1260, 1180, 1160, 1110, 1060, 1000 |
| m.p. | 89.0-91.0°C |

Example 19

8-propoxy-5-propylthioimidazo[1,2-d][1.2.4]triazine

The title compound was prepared in the same manner as described in example 7, by use of 1-chloropropane in place of 4-chlorobenzyl chloride. Yield: 47.4%.

| | |
|---|---|
| 1H-NMR($CDCl_3$) δ | 0.93 (3H,t,J=7.32Hz), 1.07 (3H,t,J=7.32Hz), 1.83-1.93 (4H,m), 3.28 (2H,t,J=7.32Hz), 4.47 (2H,t,J=7.32Hz), 7.12 (1H,s), 7.23(1H,s) |
| IRν $_{max}$ $cm^{-1}$ (KBr) | 3100, 2950, 1600, 1470, 1280, 1180, 1150, 1100 |
| m.p. | 73.5-74.5°C |

Example 20

8-butoxy-5-butylthioimidazo[1,2-d][1.2.4]triazine

The title compound was prepared in the same manner as described in example 7, by use of 1-chlorobutane in place of 4-chlorobenzyl chloride. Yield: 36.6%.

| | |
|---|---|
| 1H-NMR($CDCl_3$) δ | 0.96 (3H,t,J=7.32Hz), 0.98 (3H,t,J=7.32Hz), 1.32-1.61 (4H,m), 1.82-1.93 (4H,m), 3.32-3.56 (2H,m), 4.55 (2H,t,J=7.32Hz), 7.21(1H,s), 7.30(1H,s) |
| IRν $_{max}$ $cm^{-1}$ (KBr) | 2950, 2860, 1600, 1460, 1380, 1260, 1080, 1060 |
| m.p. | 67.5-68.0°C |

Example 21

8-pentyloxy-5-pentylthioimidazo[1,2-d][1.2.4]triazine

The title compound was prepared in the same manner as described in example 7, by use of 1-chloropentane in place of 4-chlorobenzyl chloride. Yield: 44.1%.

| | |
|---|---|
| 1H-NMR($CDCl_3$) δ | 0.86-0.94 (6H,m), 1.31-1.50 (8H,m), 1.78-1.86 (4H,m), 3.30 (2H,t,J=7.32Hz), 4.49 (2H,t,J=7.32Hz), 7.12(1H,s), 7.23(1H,s) |
| IRν $_{max}$ $cm^{-1}$ (NaCl) | 2950, 2925, 1600, 1470, 1270, 1100, 1060 |

Example 22

8-hexyloxy-5-hexylthioimidazo[1,2-d][1.2.4]triazine

The title compound was prepared in the same manner as described in example 7, by use of 1-chlorohexane in place of 4-chlorobenzyl chloride. Yield: 43.4%.

| | |
|---|---|
| 1H-NMR($CDCl_3$) δ | 0.87-0.91 (6H,m), 1.31-1.58 (12H,m), 1.77-1.86 (4H,m), 3.30 (2H,t,J=7.32Hz), 4.49 (2H,t,J=7.32Hz), 7.12 (1H,s), 7.23 (1H,s) |
| IRν $_{max}$ $cm^{-1}$ (NaCl) | 2925, 2850, 1600, 1470, 1380, 1270, 1100, 1060 |

Example 23

8-(3-chloropropoxy)-5-[(3-chloropropyl)thio]imidazo[1,2-d][1.2.4]triazine

The title compound was prepared in the same manner as described in example 7, by use of 1-bromo-3-chloropropane in place of 4-chlorobenzyl chloride. Yield: 21.8%.

| | |
|---|---|
| 1H-NMR($CDCl_3$) δ | 2.29-2.39 (4H,m), 3.46 (2H,t,J=6.84Hz), 3.54 (2H,t,J=6.10Hz), 3.71 (2H,t,J=6.10Hz), 4.68 (2H,t,J=6.84Hz), 7.21 (1H,s), 7.26 (1H,s) |
| IRν $_{max}$ $cm^{-1}$ (KBr) | 3300, 1600, 1470, 1440, 1270, 1190, 1110, 920 |
| m.p. | 71.0-72.0°C |

Example 24

8-ethoxycarbonylmethyloxy-5-ethoxy carbonylmethylthio imidazo[1,2-d][1.2.4]triazine

The title compound was prepared in the same manner as described in example 7, by use of ethyl chloroacetate in place of 4-chlorobenzyl chloride. Yield: 33.0%.

| | |
|---|---|
| 1H-NMR(DMSO-$d_6$) δ | 1.27 (6H,dt,J=7.08,7.08Hz), 4.24 (4H,q,J=7.08Hz), 5.40 (2H,s), 7.31 (1H,s), 7.65 (1H,s) |
| IRν $_{max}$ $cm^{-1}$ (KBr) | 1750, 1740, 1470, 1310, 1230 |
| m.p. | 112.0-114 °C |

Example 25

2-(4-chlorobenzyl)-4-(4-chlorobenzylthio)-8-methyl imidazo[1,5-d][1.2.4]triazine-1(2H)-one(a) and 1-(4-chlorobenzyloxy)-4-(4-chlorobenzylthio)-8-methylimidazo [1,5-d][1.2.4]triazine (b)

To the solution of 1-hydroxy-4-mercapto-8-methylimidazo[1,5-d][1.2.4]triazine (3.0g, 16.5mmol) in N,N-dimethyl formamide (60ml) 90% potassium tert-butoxide (4.6g, 38.5mmol) and 4-chlorobenzyl chloride (6.2g, 38.5mmol) were added under cooling in an ice-bath. The reaction mixture was stirred for 24h at room temperature. The reaction mixture was poured into ice-water. The precipitate was filtered, washed with water and dried to give the crude products and the products were chromatographed on silica gel eluting with hexane/ethyl acetate (5/1).

The first eluent was evaporated to give the title compound (a, 50.4%).

The second eluent was evaporated to give the title compound (b, 42.5%).

(a)

| | |
|---|---|
| 1H-NMR($CDCl_3$) δ | 2.47 (3H,s), 4.43 (2H,s), 5.54(2H,s), 7.10 (2H,d,J=8.31Hz), 7.26-7.39 (6H,m), 7.57 (1H,s) |
| IRν $_{max}$ $cm^{-1}$ (KBr) | 1620, 1500, 1480, 1410, 1300, 1220, 1080, 1020, 980 |
| m.p. | 161.0-163.0°C |

(b)

| | |
|---|---|
| 1H-NMR($CDCl_3$) δ | 2.49 (3H,s), 4.45 (2H,s), 5.10 (2H,s), 7.02 (2H,d,J=8.30Hz), 7.27-7.41 (6H,m), 7.56 (1H,s) |
| IRν $_{max}$ $cm^{-1}$ (KBr) | 1610, 1550, 1500, 1470, 1100, 1020 |
| m.p. | 161.0-163.0 °C |

Example 26

2-ethyl-4-ethylthio-8-methylimidazo[1,5-d][1.2.4]triazine-1(2H)-one(a) and 1-ethoxy-4-ethylthio-8-methylimidazo[1,5-d][1.2.4]triazine(b)

The title compound was prepared in the same manner as described in example 25, by use of 1-bromoethane in place of 4-chlorobenzyl chloride (yield; a: 22.0%, b:16.0%).

(a)

| | |
|---|---|
| 1H-NMR($CDCl_3$) δ | 1.46 (3H,t,J=7.33Hz), 1.48 (3H,t,J=7.32Hz), 2.61 (3H,s), 3.28 (2H,q,J=7.33Hz), 3.97 (2H,q,J=7.33Hz), 7.52(1H,s) |
| IRν $_{max}$ $cm^{-1}$ (NaCl) | 2975, 2925, 1610, 1480, 1370, 1150, 1040 |
| MS(EI) | 254 ($M^+$) |

(b)

| | |
|---|---|
| 1H-NMR($CDCl_3$) δ | 1.45 (3H,t,J=7.08Hz) 1.52 (3H,t,J=7.32Hz), 2.50 (3H,s), 3.30 (2H,q,J=7.32Hz), 4.40 (2H,q,J=7.08Hz), 7.51(1H,s) |
| IRν $_{max}$ $cm^{-1}$ (KBr) | 3100, 2975, 2925, 1610, 1480, 1280, 1200, 1180, 1080 |
| m.p. | 79.0-80.5°C |

Example 27

5-(4-chlorobenzylthio)-8-ethoxycarbonylmethyloxyimidazo [1,2-d][1.2.4]triazine

To the solution of 5-(4-chlorobenzylthio)-8-hydroxy imidazo[1,2-d][1.2.4]triazine (Example 1, 1.5g, 5.5mmol) in N,N-dimethyl formamide (30ml) 90% potassium tert-butoxide (0.98g, 7.9mmol) and ethyl bromoacetate (1.05g, 6.3mmol) were added under cooling in an ice-bath. The reaction mixture was stirred for 20h at room temperature. The reaction mixture was poured into ice-water. The precipitate was filtered, washed with water and dried to give the title compound (0.8g, 40.2%).

| | |
|---|---|
| 1H-NMR($CDCl_3$) δ | 1.27 (3H,t,J=7.08Hz), 4.24 (2H,q,J=7.08Hz), 4.74 (2H,s), 5.29 (2H,s), 7.13(1H,d,J=0.98Hz), 7.28-7.40 (5H,m) |
| IRν $_{max}$ $cm^{-1}$ (KBr) | 1760, 1740, 1480, 1240, 1100 |
| m.p. | 126.5-128.0°C |

Example 28

4-(4-chlorobenzylthio)-2-ethoxycarbonylmethyl-8-methyl imidazo[1,5-d][1.2.4]triazine-1(2H)-one(a) and 4-(4-chlorobenzylthio)-1-ethoxycarbonylmethyloxy-8-methyl imidazo[1,5-d][1.2.4]triazine(b)

The title compound was prepared in the same manner as described in example 27, by use of 4-(4-chlorobenzylthio)-1-hydroxy-8-methylimidazo[1,2-d][1.2.4]triazine (Example 5) in place of 5-(4-chlorobenzylthio)-8-hydroxyimidazo[1,2-d][1.2.4]triazine (yield; a: 34.0%, b: 20.6%).

(a)

| | |
|---|---|
| 1H-NMR($CDCl_3$) δ | 1.30 (3H,t,J=7.08Hz), 2.55 (3H,s), 4.27 (2H,q,J=7.08Hz), 4.45 (2H,s), 4.67 (2H,s), 7.28 (2H,d,J=8.55Hz), 7.39 (2H,d,J=8.30Hz), 7.53 (1H,s) |
| IRν $_{max}$ $cm^{-1}$ (KBr) | 1740, 1620, 1500, 1480, 1380, 1360, 1220, 1160, 1100, 1030 |
| m.p. | 118.0-119.0°C |

(b)

| | |
|---|---|
| 1H-NMR($CDCl_3$) δ | 1.26 (3H,t,J=7.08Hz), 2.49 (3H,s), 4.29 (2H,q,J=7.08Hz), 4.44 (2H,s), 5.11 (2H,s), 7.29-7.40 (2H,m), 7.53 (1H,s) |
| IRν $_{max}$ $cm^{-1}$ (KBr) | 1740, 1620, 1500, 1480, 1380, 1230, 1080, 1030, 1020 |
| m.p. | 139.0-146.0°C |

Example 29

4-(4-chlorobenzylsulfinyl)-1-hydroxypyrrolo[1,2-d][1.2.4]triazine

To the solution of 4-(4-chlorobenzylthio)-1-hydroxy pyrrolo[1,2-d][1,2,4]triazine (Example 3, 0.3g, 1mmol) in dichloromethane (10ml) 80% p-chloroperbenzoic acid (0.25g, 1.2mmol) was added under cooling in an ice-bath. The reaction mixture was stirred for 2h at the same temperature. The reaction mixture was poured into saturated aqueous sodium hydrogen carbonate and extracted with chloroform. The extract was washed with water, dried and evaporated. The residue was dissolved in ethanol and the solution was treated with Norit. The solution was evaporated and the residue was recrstallized from benzene/n-hexane to give the title compound (0.2g, 65.6%).

| | |
|---|---|
| 1H-NMR($CDCl_3$) δ | 4.61 (2H,d,J=5.37Hz), 6.37-6.41 (1H,m), 6.98-7.35 (6H,m), 7.36 (1H,s) |
| IRν $_{max}$ $cm^{-1}$ (KBr) | 3400, 3200, 1610, 1510, 1485, 1400, 1125, 1090, 1065 |
| m.p. | 138.0-140.0°C |

Example 30

4-(4-fluorobenzylsulfinyl)-1-hydroxypyrrolo[1,2-d][1.2.4]triazine

The title compound was prepared in the same manner as described in example 29, by use of 4-(4-fluorobenzylthio)-1-hydroxypyrrolo[1,2-d][1.2.4]triazine (Example 4) in place of 4-(4-chlorobenzylthio)-1-hydroxypyrrolo[1,2-d][1.2.4] triazine (Example 3). Yield: 35.4%.

| | |
|---|---|
| 1H-NMR($CDCl_3$) δ | 4.61 (2H,d,J=5.37Hz), 6.37-6.41 (1H,m), 6.98-7.11 (4H,m), 7.28-7.39 (2H,m), 9.61(1H,brs) |
| IRν $_{max}$ $cm^{-1}$ (KBr) | 3450, 3080, 1610, 1510, 1395, 1220, 1160, 1130, 1060 |
| m.p. | 155.5-157.0°C |

Example 31

5-(4-chlorobenzylsulfinyl)-8-hydroxyimidazo[1,2-d][1.2.4]triazine

The title compound was prepared in the same manner as described in example 29, by use of 5-(4-chlorobenzylthio)-8-hydroxyimidazo[1,2-d][1.2.4]triazine (Example 1) in place of 4-(4-chlorobenzylthio)-1-hydroxypyrrolo[1,2-d][1.2.4] triazine (Example 3). Yield: 84.9%.

| | |
|---|---|
| 1H-NMR($CDCl_3$/$CD_3OD_3$=5/1) δ | 4.68 (2H,d,J=2.93Hz), 7.24-7.35 (6H,m) |
| IRν $_{max}$ $cm^{-1}$ (KBr) | 3450, 1615, 1520, 1485, 1380, 1130, 1100, 1065, 1000 |
| m.p. | 177.5-179.0°C |

Example 32

8-hydroxy-5-(3-phenylpropylsulfinyl) imidazo [1,2-d][1.2.4]triazine

The title compound was prepared in the same manner as described in example 29, by use of 8-hydroxy-5-(3-phenylpropylthio)imidazo[1,2-d][1.2.4]triazine (Example 2) in place of 4-(4-chlorobenzylthio)-1-hydroxypyrrolo[1,2-d][1.2.4]triazine (Example 3). Yield: 89.4%.

| | |
|---|---|
| 1H-NMR($CDCl_3$) δ | 2.11-2.28 (2H,m), 2.82 (2H,t,J=7.32Hz), 3.30-3.52 (2H,m), 7.15-7.30 (5H,m), 7.43 (2H,d,J=7.33Hz) |
| IRν $_{max}$ $cm^{-1}$ (KBr) | 2800, 1630, 1530, 1500, 1450, 1400, 1070, 1010 |
| m.p. | 161.0-166.0°C |

Example 33

4-(4-chlorobenzylsulfinyl)-1-hydroxy-8-methyl imidazo [1,5-d][1.2.4]triazine

The title compound was prepared in the same manner as described in example 29, by use of 4-(4-chlorobenzylthio)-1-hydroxy-8-methylimidazo[1,5-d][1.2.4]triazine (Example 5) in place of 4-(4-chlorobenzylthio)-1-hydroxypyrrolo[1,2-d][1.2.4]triazine (Example 3). Yield: 67.8%.

| | |
|---|---|
| 1H-NMR($CDCl_3$) δ | 2.51 (3H,s), 4.79. (2H,dd,J=17.13,12.69Hz), 7.34 (2H,d,J=8.30Hz), 7.42 (2H,d,J=8.30Hz), 7.79 (1H,s) |
| IRν $_{max}$ $cm^{-1}$ (KBr) | 3400, 3100, 2910, 2850, 1610, 1565, 1490, 1405, 1320, 1090, 1060, 1015, 945 |
| m.p. | 168.0-170.0°C |

Example 34

1-hydroxy-8-methyl-4-(3-phenylpropylsulfinyl)imidazo[1,5-d][1.2.4]triazine

The title compound was prepared in the same manner as described in example 29, by use of 1-hydroxy-8-methyl-4-(3-phenylpropylthio)imidazo[1,5-d][1.2.4]triazine (Example 6) in place of 4-(4-chlorobenzylthio)-1-hydroxypyrrolo[1,2-d][1.2.4]triazine (Example 3). Yield: 28.2%.

| | |
|---|---|
| 1H-NMR($CDCl_3$) δ | 2.11-2.29 (2H,m), 2.62 (3H,s), 2.80-2.85 (2H,m), 3.30-3.53 (2H,m), 7.17-7.31 (4H,m), 7.52 (1H,s), 7.58(1H,s) |
| IRν $_{max}$ $cm^{-1}$ (KBr) | 3100, 1600, 1570, 1460, 1350, 1320, 1260, 1060, 950 |
| m.p. | 170.0-172.0°C |

Example 35

8-( 4-chlorobenzyloxy)-5-(4-chlorobenzylsulfinyl)imidazo[1,2-d][1.2.4]triazine

The title compound was prepared in the same manner as described in example 29, by use of 8-(4-chlorobenzyloxy)-5-(4-chlorobenzylthio)imidazo[1,2-d][1.2.4]triazine (Example 7) in place of 4-(4-chlorobenzylthio)-1-hydroxypyrrolo[1,2-d][1.2.4]triazine (Example 3). Yield: 78.0%.

| | |
|---|---|
| 1H-NMR($CDCl_3$) δ | 4.63 (2H,s), 5.73 (2H,s), 7.15-7.35 (10H,m), |
| IRν $_{max}$ $cm^{-1}$ (KBr) | 3430, 1595, 1485, 1410, 1275, 1090, 1070, 1010 |
| m.p. | 161.0-162.0°C |

Example 36

1-(4-chlorobenzyloxy)-4-(4-chlorobenzylsulfinyl)pyrrolo [1,2-d][1.2.4]triazine

The title compound was prepared in the same manner as described in example 29, by use of 8-(4-chlorobenzyloxy)-5-(4-chlorobenzylthio)imidazo[1,2-d][1.2.4]triazine (Example 8) in place of 4-(4-chlorobenzylthio)-1-hydroxypyrrolo[1,2-d][1.2.4]triazine (Example 3). Yield: 72.0%.

| | |
|---|---|
| 1H-NMR($CDCl_3$) δ | 5.37 (2H,d,J=4.56Hz), 5.64 (2H,s), 6.34 (2H,dd,J=3.90,2.93Hz), 6.99 (2H,d,J=3.90Hz), 7.05 (2H,d,J=8.55Hz), 7.19 (2H,d,J=8.54Hz), 7.28 (2H,d,J=8.54Hz), 7.29 (2H,d,J=8.55Hz) |
| IRν $_{max}$ $cm^{-1}$ (KBr) | 1600, 1490, 1080, 1090, 1010 |
| m.p. | 138.0-139.0°C |

Example 37

8-(4-methylbenzyloxy)-5-(4-metylbenzylsulfinyl)imidazo [1,2-d][1.2.4]triazine

The title compound was prepared in the same manner as described in example 29, by use of 8-(4-methylbenzyloxy)-5-(4-metylbenzylthio)imidazo[1,2-d][1.2.4]triazine (Example 9) in place of 4-(4-chlorobenzylthio)-1-hydroxypyrrolo[1,2-d][1.2.4]triazine (Example 3). Yield: 70.0%.

| | |
|---|---|
| 1H-NMR($CDCl_3$) δ | 2.30 (3H,s), 2.33 (3H,s), 4.64 (2H,d,J=0.97Hz), 5.70 (2H,s), 7.08-7.17 (9H,m), 7.32 (1H,d,J=0.97Hz) |
| IRν $_{max}$ $cm^{-1}$ (KBr) | 2900, 1600, 1520, 1480, 1460, 1280, 1080 |
| m.p. | 119.0-126.0°C |

Example 38

8-(4-isopropylbenzyloxy)-5-(4-isopropylbenzylsulfinyl) imidazo[1,2-d][1,2,4]triazine

The title compound was prepared in the same manner as described in example 29, by use of 8-(4-isopropylbenzyloxy)-5-(4-isopropylbenzylthio)imidazo[1,2-d][1.2.4]triazine (Example 10) in place of 4-(4-chlorobenzylthio)-1-hydroxypyrrolo[1,2-d][1.2.4]triazine (Example 3). Yield: 93.0%.

| | |
|---|---|
| 1H-NMR($CDCl_3$) δ | 1.17-1.24 (12H,m), 2.81-2.94 (2H,m), 4.65 (2H,s), 5.72 (2H,s), 7.17-7.31 (10H,m) |
| IRν $_{max}$ $cm^{-1}$ (NaCl) | 2950, 1600, 1520, 1490, 1460, 1420, 1260, 1060 |
| MS(EI) | 448(M+) |

Example 39

8-(4-tert-butylbenzyloxy)-5-(4-tert-butylbenzylsulfinyl) imidazo[1,2-d][1.2.4]triazine

The title compound was prepared in the same manner as described in example 29, by use of 8-(4-tert-butylbenzyloxy)-5-(4-tert-butylbenzylthio)imidazo[1,2-d][1.2.4]triazine (Example 11) in place of 4-(4-chlorobenzylthio)-1-hydroxypyrrolo[1,2-d][1.2.4]triazine (Example 3). Yield: 36.5%.

| | |
|---|---|
| 1H-NMR($CDCl_3$) δ | 1.28 (9H,d,J=3.17Hz), 1.30 (9H,d,J=3.17Hz), 4.65 (2H,s), 5.73 (2H,s), 7.18-7.27 (5H,m), 7.31-7.38 (5H,m) |
| IRν $_{max}$ $cm^{-1}$ (KBr) | 2950, 1600, 1460, 1360, 1260, 1100, 1050 |
| m.p. | 166.0-168.0°C |

Example 40

8-(3-chlorobenzyloxy)-5-(3-chlorobenzylsulfinyl)imidazo[1,2-d][1.2.4]triazine

The title compound was prepared in the same manner as described in example 29, by use of 8-(3-chlorobenzyloxy)-5-(3-chlorobenzylthio)imidazo[1,2-d][1.2.4]triazine (Example 14) in place of 4-(4-chlorobenzylthio)-1-hydroxypyrrolo[1,2-d][1.2.4]triazine (Example 3). Yield: 46.0%.

| | |
|---|---|
| 1H-NMR($CDCl_3$) δ | 4.66 (2H,t,J=13.43Hz), 5.68 (2H,t,J=13.43Hz), 7.03-7.29 (10H,m) |
| IRν $_{max}$ $cm^{-1}$ (KBr) | 3000, 1600, 1480, 1430, 1220, 1090 |
| m.p. | 110.0-112.0°C |

Example 41

8-(4-cyanobenzyloxy)-5-(4-cyanobenzylsulfinyl)imidazo[1,2-d][1.2.4]triazine

The title compound was prepared in the same manner as described in example 29, by use of 8-(3-chlorobenzyloxy)-5-(3-chlorobenzylthio)imidazo[1,2-d][1.2.4]triazine (Example 14) in place of 4-(4-chlorobenzylthio)-1-hydroxypyrrolo[1,2-d][1.2.4]triazine (Example 3). Yield: 82.0%.

| | |
|---|---|
| 1H-NMR($CDCl_3$) δ | 4.89 (2H,dd,J=12.45,24.90Hz), 5.86(2H,s), 7.37 (3H,d,J=7.08Hz), 7.50 (2H,d,J=8.06Hz), 7.81-7.84 (5H,m) |
| IRν $_{max}$ $cm^{-1}$ (KBr) | 3430, 2220, 1600, 1500, 1460, 1420 |
| m.p. | 179.0-180.0°C |

Example 42

8-(4-trifluoromethylbenzyloxy)-5-(4-trifluoromethylbenzyl sulfinyl)imidazo[1,2-d][1.2.4]triazine

The title compound was prepared in the same manner as described in example 29, by use of 8-(4-triflu oromethylbenzyloxy)-5-(4-trifluoromethylbenzylthio)imidazo[1,2-d][1.2.4]triazine (Example 13 ) in place of 4-(4-chlorobenzylthio)-1-hydroxy pyrrolo[1,2-d][1.2.4]triazine (Example 3). Yield: 85.0%.

| | |
|---|---|
| 1H-NMR($CDCl_3$) δ | 4.71 (2H,s), 5.84 (2H,s), 7.24 (2H,d,J=7.56Hz), 7.32 (2H,d,J=8.06Hz), 7.38(1H,s), 7.45 (2H,d,J=8.06Hz), 7.58-7.63 (4H,m) |
| IRν $_{max}$ $cm^{-1}$ (KBr) | 3430, 1600, 1420, 1330, 1170, 1120, 1070, 1020 |
| m.p. | 116.0-117.0°C |

Example 43

8-(4-phenylbenzyloxy)-5-(4-phenylbenzylsulfinyl)imidazo[1,2-d][1.2.4]triazine

The title compound was prepared in the same manner as described in example 29, by use of 8-(4-phenylbenzyloxy)-5-(4-phenylbenzylthio)imidazo[1,2-d][1.2.4]triazine (Example 15) in place of 4-(4-chlorobenzylthio)-1-hydroxypyrrolo[1,2-d][1.2.4]triazine (Example 3). Yield: 78.2%.

| | |
|---|---|
| 1H-NMR($CDCl_3$) δ | 4.72 (2H,s), 5.80 (2H,s), 7.22-7.64 (20H,m) |
| IRν $_{max}$ $cm^{-1}$ (KBr) | 1600, 1490, 1465, 1410, 1265, 1090, 1065 |
| m.p. | 106.0-107.0°C (d) |

Example 44

8-phenethyloxy-5-phenethylsulfinylimidazo[1,2-d][1.2.4]triazine

The title compound was prepared in the same manner as described in example 29, by use of 8-phenethyloxy-5-phenethylthioimidazo[1,2-d][1.2.4]triazine (Example 16) in place of 4-(4-chlorobenzylthio)-1-hydroxypyrrolo[1,2-d][1.2.4]triazine (Example 3). Yield: 84.8%.

| | |
|---|---|
| 1H-NMR($CDCl_3$) δ | 3.13 (2H,t,J=7.08Hz), 3.18-3.32 (2H,m), 3.56-3.85 (2H,m), 4.76 (2H,t,J=7.08Hz), 6.96 (1H,d,J=0.73Hz), 7.09-7.36(11H,m) |
| IRν $_{max}$ $cm^{-1}$ (KBr) | 3025, 2925, 1585, 1490, 1455, 1350, 1265, 1145, 1195, 1090, 1060 |
| m.p. | 84.0-85.0°C |

Example 45

8-(3-phenylpropoxy)-5-(3-phenylpropyl)sulfinylimidazo[1,2-d][1.2.4]triazine

The title compound was prepared in the same manner as described in example 29, by use of 8-(3-phenylpropoxy)-5-(3-phenylpropylthio)imidazo[1,2-d][1.2.4]triazine (Example 17) in place of 4-(4-chlorobenzylthio)-1-hydroxypyrrolo[1,2-d][1.2.4]triazine (Example 3). Yield: 84.9%.

| | |
|---|---|
| 1H-NMR($CDCl_3$) δ | 2.19-2.30 (4H,m), 2.70 (2H,t,J=7.57Hz), 2.84 (2H,t,J=7.57Hz), 3.30-3.41(1H,m), 3.44-3.55 (1H,m), 4.56 (2H,t,J=7.32Hz), 7.14-7.38 (12H,m) |
| IRν $_{max}$ $cm^{-1}$ (KBr) | 3075, 2925, 1600, 1490, 1460, 1450, 1260, 1080, 920 |
| m.p. | 82.5-84.0°C |

Example 46

8-ethoxy-5-ethylsulfinylimidazo[1,2-d][1.2.4]triazine

The title compound was prepared in the same manner as described in example 29, by use of 8-ethoxy-5-ethylthioimidazo[1,2-d][1.2.4]triazine (Example 18) in place of 4-(4-chlorobenzylthio)-1-hydroxypyrrolo[1,2-d][1.2.4]triazine (Example 3). Yield: 92.0%.

| | |
|---|---|
| 1H-NMR($CDCl_3$) δ | 1.45 (3H,t,J=7.33Hz), 1.52 (3H,t,J=7.32Hz), 3.47 (2H,q,J=7.33Hz), 4.60 (2H,q,J=7.32Hz), 7.24(1H,s), 7.31(1H,s) |
| IRν $_{max}$ $cm^{-1}$ (KBr) | 3100, 2975, 2925, 1600, 1500, 1460, 1380, 1270, 1080 |
| m.p. | 79.0-81.5°C |

Example 47

8-propoxy-5-propylsulfinylimidazo[1,2-d][1.2.4]triazine

The title compound was prepared in the same manner as described in example 29, by use of 8-propoxy-5-propylthioimidazo [1,2-d][1.2.4]triazine (Example 19) in place of 4-(4-chlorobenzylthio)-1-hydroxypyrrolo[1,2-d][1.2.4]triazine (Example 3). Yield: 70.0%.

| | |
|---|---|
| 1H-NMR($CDCl_3$) δ | 0.98 (3H,t,J=7.33Hz), 1.15 (3H,t,J=7.33Hz), 1.84-2.02 (4H,m), 3.29-3.55 (2H,m), 4.52 (2H,t,J=7.33Hz), 7.20 (1H,s), 7.31 (1H,s) |
| IRν $_{max}$ $cm^{-1}$ (KBr) | 3100, 2950, 1600, 1490, 1460, 1380, 1350, 1260, 1150, 1080 |
| m.p. | 73.5-74.5°C |

Example 48

8-butoxy-5-butylsulfinylimidazo[1,2-d][1.2.4]triazine

The title compound was prepared in the same manner as described in example 29, by use of 8-butoxy-5-butylthioimidazo[1,2-d][1.2.4]triazine (Example 20) in place of 4-(4-chlorobenzylthio)-1-hydroxypyrrolo[1,2-d][1.2.4]triazine (Example 3). Yield: 90.7%.

| | |
|---|---|
| 1H-NMR($CDCl_3$) δ | 0.96 (3H,t,J=7.32Hz), 0.98 (3H,t,J=7.32Hz), 1.32-1.61 (4H,m), 1.82-1.93 (4H,m), 3.32-3.56 (2H,m), 4.55 (2H,t,J=7.32Hz), 7.21 (1H,s), 7.30 (1H,s) |
| IR$\nu_{max}$ $cm^{-1}$ (NaCl) | 2950, 2860, 1600, 1460, 1380, 1260, 1080, 1060 |
| MS(EI) | 296(M+) |

Example 49

8-pentyloxy-5-pentylsulfinylimidazo[1,2-d][1.2.4]triazine

The title compound was prepared in the same manner as described in example 29, by use of 8-pentyloxy-5-pentylthio imidazo[1,2-d][1,2,4]triazine (Example 21) in place of 4-(4-chlorobenzylthio)-1-hydroxypyrrolo[1,2-d][1,2,4]triazine (Example 3). Yield: 93.0%.

| | |
|---|---|
| 1H-NMR($CDCl_3$) δ | 0.88-0.94 (6H,m), 1.32-1.55(8H,m), 1.79-1.99 (4H,m), 3.31-3.55 (2H,m), 4.54 (2H,t,J=7.08Hz), 7.20 (1H,s), 7.32 (1H,s) |
| IR$\nu_{max}$ $cm^{-1}$ (NaCl) | 2950, 2850, 1710, 1590, 1460, 1350, 1260, 1140, 1090, 1060 |
| MS(EI) | 324(M+) |

Example 50

8-hexyloxy-5-hexylsulfinylimidazo[1,2-d][1.2.4]triazine

The title compound was prepared in the same manner as described in example 29, by use of 8-hexyloxy-5-hexylthioimidazo[1,2-d][1.2.4]triazine (Example 22) in place of 4-(4-chlorobenzylthio)-1-hydroxypyrrolo[1,2-d][1.2.4]triazine (Example 3). Yield: 95.2%.

| | |
|---|---|
| 1H-NMR($CDCl_3$) δ | 0.72-0.89 (6H,m), 1.32-1.51 (12H,m), 1.83-1.91 (4H,m), 3.31-3.55(2H,m), 4.54 (2H,t,J=7.32Hz), 7.20(1H,s), 7.30 (1H,s) |
| IR$\nu_{max}$ $cm^{-1}$ (NaCl) | 2950, 2925, 2850, 1710, 1590, 1460, 1380, 1260, 1090, 1060 |
| MS(EI) | 352(M+) |

Example 51

8-(3-chloropropoxy)-5-(3-chloropropyl)sulfinylimidazo[1,2-d][1.2.4]triazine

The title compound was prepared in the same manner as described in example 29, by use of 8-(3-chloropropoxy)-5-[(3-chloropropyl)thio]imidazo[1,2-d][1.2.4]triazine (Example 23) in place of 4-(4-chlorobenzylthio)-1-hydroxypyrrolo[1,2-d][1.2.4]triazine (Example 3). Yield: 65.2%.

| | |
|---|---|
| 1H-NMR($CDCl_3$) δ | 2.31-2.49 (4H,m), 3.53-3.65(4H,m), 3.73(2H,t,J=5.62Hz), 4.74(2H,t,J=6.84Hz), 7.20(1H,s), 7.32(1H,s) |
| IR$\nu_{max}$ $cm^{-1}$ (KBr) | 1600, 1480, 1460, 1270, 1100, 1080 |
| m.p. | 56.0-57.5°C |

Example 52

8-ethoxycarbonylmethyloxy-5-ethoxycarbonylmeth ylsulfinylimidazo[1,2-d][1.2.4]triazine

The title compound was prepared in the same manner as described in example 29, by use of 8-ethoxycarbonylmethyloxy-5-ethoxycarbonylmethylthioimidazo[1,2-d][1.2.4]triazine (Example 24) in place of 4-(4-chlorobenzylthio)-1-hydroxypyrrolo[1,2-d][1.2.4]triazine (Example 3). Yield: 62.1%.

| | |
|---|---|
| 1H-NMR($CDCl_3$) δ | 1.19 (3H,t,J=7.08Hz), 1.20 (3H,t,J=7.08Hz), 4.16 (4H,q,J=7.08Hz), 4.38(2H,dd,J=31.74,31.74Hz), 5.25 (2H,s), 7.16 (1H,s), 7.28 (1H,s) |
| IRν $_{max}$ $cm^{-1}$ (NaCl) | 1740, 1600, 1470, 1300, 1220, 1100, 1020 |
| MS(EI) | 311($M^+$ -1) |

Example 53

1-(4-chlorobenzyloxy)-4-(4-chlorobenzylsulfinyl)-8-methylimidazo[1,5-d][1.2.4]triazine

The title compound was prepared in the same manner as described in example 29, by use of 1-(4-chlorobenzyloxy)-4-(4-chlorobenzylthio)-8-methylimidazo[1,2-d][1.2.4]triazine (Example 25(b)) in place of 4-(4-chlorobenzylthio)-1-hydroxypyrrolo[1,2-d][1.2.4]triazine (Example 3). Yield: 93.3%.

| | |
|---|---|
| 1H-NMR($CDCl_3$) δ | 2.42 (3H,s), 4.43 (2H,s), 5.54 (2H,s), 7.10 (2H,d,J=8.31Hz), 7.26-7.39 (6H,m), 7.57 (1H,s) |
| IRν $_{max}$ $cm^{-1}$ (KBr) | 1620, 1500, 1480, 1410, 1300, 1220, 1080, 1020, 980 |
| m.p. | 138.0-140.0°C |

Example 54

2-(4-chlorobenzyl)-4-(4-chlorobenzylsulfinyl)-8-methyl imidazo[1,5-d][1.2.4]triazine-1(2H)-one

The title compound was prepared in the same manner as described in example 29, by use of 2-(4-chlorobenzyl)-4-(4-chlorobenzylthio)-8-methylimidazo[1,5-d][1.2.4]triazine -1(2H)-one (Example 25(a)) in place of 4-(4-chlorobenzylthio)-1-hydroxypyrrolo[1,2-d][1.2.4]triazine (Example 3). Yield: 94.2%.

| | |
|---|---|
| 1H-NMR($CDCl_3$) δ | 2.50 (3H,s), 4.63 (2H,d,J=3.90Hz), 5.18(2H,s), 7.04 (2H,d,J=6.35Hz), 7.27-7.38 (7H,m) |
| IRν $_{max}$ $cm^{-1}$ (KBr) | 1780, 1610, 1540, 1500, 1360, 1140, 1100, 1020 |
| m.p. | 83.0-86.0 °C |

Example 55

1-ethoxy-4-ethylsulfinyl-8-methylimidazo[1,5-d][1.2.4]triazine

The title compound was prepared in the same manner as described in example 29, by use of 1-ethoxy-4-ethylthio-8-methylimidazo[1,5-d][1.2.4]triazine (Example 26(b)) in place of 4-(4-chlorobenzylthio)-1-hydroxypyrrolo[1,2-d][1.2.4]triazine (Example 3). Yield: 92.5%.

| | |
|---|---|
| 1H-NMR($CDCl_3$) δ | 1.48 (3H,t,J=7.32Hz), 1.49 (3H,t,J=7.57Hz), 2.58 (3H,s), 3.47 (2H,q,J=7.57Hz), 4.45 (2H,q,J=7.33Hz), 7.61 (1H,s) |
| IRν $_{max}$ $cm^{-1}$ (KBr) | 3000, 2950, 1610, 1500, 1380, 1320, 1200, 1080 |
| m.p. | 79.0-80.5°C |

Example 56

2-ethyl-4-ethylsulfinyl-8-methylimidazo[1,5-d][1.2.4]triazine-1(2H)-one

The title compound was prepared in the same manner as described in example 29, by use of 2-ethyl-4-ethylthio-8-ethyl imidazo[1,5-d][1.2.4]triazine-1(2H)-one (Example 26(a)) in place of 4-(4-chlorobenzylthio)-1-hydroxypyrrolo[1,2-d][1.2.4]triazine (Example 3). Yield: 91.5%.

| | |
|---|---|
| 1H-NMR($CDCl_3$) δ | 1.36 (3H,t,J=7.32Hz), 1.41 (3H,t,J=7.32Hz), 2.58 (3H,s), 3.37 (2H,q,J=7.32Hz), 3.93 (2H,q,J=7.32Hz), 7.51(1H,s) |
| IRν $_{max}$ $cm^{-1}$ (NaCl) | 3000, 2950, 1610, 1550, 1450, 1380, 1350, 1250, 1080, 1050 |

Example 57

5-(4-chlorobenzylsulfinyl)-8-ethoxycarbonylmet hyloxyimidazo[1,2-d][1.2.4]triazine

The title compound was prepared in the same manner as described in example 29, by use of 5-(4-chlorobenzylthio)-8-ethoxycarbonylmethyloxyimidazo[1,2-d][1.2.4]triazine (Example 27) in place of 4-(4-chlorobenzylthio)-1-hydroxy pyrrolo[1,2-d][1.2.4]triazine (Example 3). Yield: 62.1%.

| | |
|---|---|
| 1H-NMR($CDCl_3$) δ | 1.28 (3H,t,J=7.08Hz), 4.25 (2H,q,J=7.08Hz), 4.63 (2H,s), 5.30 (2H,s), 7.21-7.44 (6H,m) |
| IR$\nu_{max}$ $cm^{-1}$ (KBr) | 1760, 1740, 1600, 1490, 1240, 1100, 1080 |
| m.p. | 154.0-156.0°C |

Example 58

4-(4-chlorobenzylsulfinyl)-1-(ethoxycarbonylmethyloxy)-8-methylimidazo[1,5-d][1.2.4]triazine

The title compound was prepared in the same manner as described in example 29, by use of 4-(4-chlorobenzylthio)-1-(ethoxycarbonylmethyloxy)-8-methylimidazo[1,5-d][1.2.4]triazine (Example 28(b)) in place of 4-(4-chlorobenzylthio)-1-hydroxypyrrolo[1,2-d][1.2.4]triazine (Example 3). Yield: 93.0%.

| | |
|---|---|
| 1H-NMR($CDCl_3$) δ | 1.19 (3H,t,J=7.08Hz), 2.42(3H,s), 4.15 (2H,q,J=7.08Hz), 4.48(1H,d,J=23.20Hz), 4.53 (1H,d,J=23.19Hz), 7.11-7.30 (4H,m), 7.55 (1H,s) |
| IR$\nu_{max}$ $cm^{-1}$ (KBr) | 1750, 1610, 1500, 1380, 1230, 1100, 1080, 1020 |
| m.p. | 163.0-167.0°C |

Example 59

2-ethoxycarbonylmethyl-4-(4-chlorobenzylsulfinyl)-8-methylimidazo[1,5-d][1.2.4]triazine-1(2H)-one

The title compound was prepared in the same manner as described in example 29, by use of 2-ethoxycarbonylmethyl-4-(4-chlorobenzylthio)-8-methylimidazo[1,5-d][1.2.4]triazine-1(2H)-one (Example 28(a)) in place of 4-(4-chlorobenzylthio)-1-hydroxypyrrolo[1,2-d][1.2.4]triazine (Example 3). Yield: 91.0%.

| | |
|---|---|
| 1H-NMR($CDCl_3$) δ | 1.31 (3H,t,J=7.08Hz), 2.57 (3H,s), 4.29 (2H,q,J=7.08Hz), 4.69 (2H,s), 4.74(2H,s), 7.26-7.33 (4H,m), 7.53 (1H,s) |
| IR$\nu_{max}$ $cm^{-1}$ (KBr) | 1740, 1620, 1500, 1480, 1380, 1360, 1220, 1160, 1100, 1030 |
| m.p. | 118.0-119.0°C |

**Claims**

1. A compound represented by the following general formula:

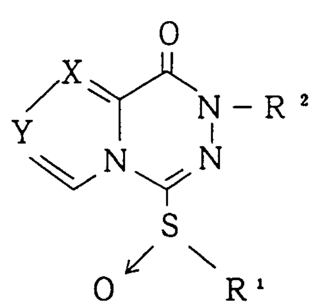

(1 a)

or

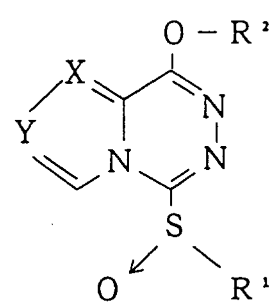

(1 b)

where in formula (1a) X is C-$CH_3$ and Y is N, $R^1$ is a lower alkyl group or a benzyl group substituted by one halogen

atom and $R^2$ is a lower alkyl group, a benzyl group substituted by one halogen atom or a lower alkoxycarbonylmethyl group, and where in formula (1b) X is N and Y is CH, X is CH and Y is CH, or X is $C\text{-}CH_3$ and Y is N, $R^1$ is a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group, and $R_2$ is a hydrogen atom, a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group.

**2.** The compound according to claim 1, which is:
8-(4-chlorobenzyloxy)-5-(4-chlorobenzylsulfinyl)imidazo[1,2-d][1.2.4]triazine,
2-ethoxycarbonylmethyl-4-(4-chlorobenzylsulfinyl)-8-methylimidazo[1,5-d][1.2.4]triazine-1(2H)-one,
8-(3-chlorobenzyloxy)-5-(3-chlorobenzylsulfinyl)imidazo[1,2-d][1.2.4]triazine,
2-(4-chlorobenzyl)-4-(4-chlorobenzylsulfinyl)-8-methylimidazo[1,5-d][1.2.4]triazine-1(2H)-one,
5-(4-chlorobenzylsulfinyl)-8-ethoxycarbonylmethyloxyimidazo[1,2-d][1.2.4]triazine,
5-(4-chlorobenzylsulfinyl)-8-hydroxyimidazo[1,2-d][1.2.4]triazine,
1-(4-chlorobenzyloxy)-4-(4-chlorobenzylsulfinyl)-8-methylimidazo[1,5-d][1.2.4]triazine,
4-(4-chlorobenzylsulfinyl)-1-(ethoxycarbonylmethyloxy)-8-methylimidazo[1,5-d][1.2.4]triazine,
8-(4-methylbenzyloxy)-5-(4-methylbenzylsulfinyl)imidazo[1,2-d][1.2.4]triazine,
8-hydroxy-5-(3-phenylpropylsulfinyl)imidazo[1,2-d][1.2.4]triazine,
8-pentyloxy-5-pentylsulfinylimidazo[1,2-d][1.2.4]triazine,
4-(4-chlorobenzylsulfinyl)-1-hydroxypyrrolo[1,2-d][1.2.4]triazine,
1-(4-chlorobenzyloxy)-4-(4-chlorobenzylsulfinyl)pyrrolo[1,2-d][1.2.4]triazine,
4-(4-chlorobenzylsulfinyl)-1-hydroxy-8-methylimidazo[1,5-d][1.2.4]triazine,
8-phenethyloxy-5-phenethylsulfinylimidazo[1,2-d][1.2.4]triazine,
8-(3-phenylpropyloxy)-5-(3-phenylpropylsulfinyl)imidazo[1,2-d][1.2.4]triazine,
1-ethoxy-4-ethylsulfinyl-8-methylimidazo[1,5-d][1.2.4]triazine,
8-ethoxy-5-ethylsulfinylimidazo[1,2-d][1.2.4]triazine, or
2-ethyl-4-ethylsulfinyl-8-methylimidazo[1,5-d][1.2.4]triazine-1(2H)-one.

**3.** A compound represented by the following general formula:

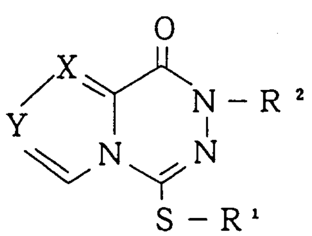

(2 a)

or

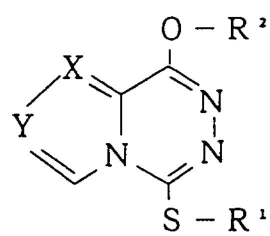

(2 b)

where in formula (2a) X is $C\text{-}CH_3$ and Y is N, $R^1$ is a lower alkyl grow or a benzyl group substituted by one halogen atom and $R^2$ is a lower alkyl group, a benzyl group substituted by one halogen atom or a lower alkoxycarbonylmethyl group, and where in formula (2b) X is N and Y is CH, X is CH and Y is CH, or X is $C\text{-}CH_3$ and Y is N, $R^1$ is a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group, and $R^2$ is a hydrogen atom, a lower alkyl group, a lower alkoxycarbonylmethyl group, a phenyl lower alkyl group or a benzyl group whose phenyl ring is substituted by any one of a lower alkyl group, a halogen atom, a cyano group, a phenyl group and a halo lower alkyl group.

**4.** A pharmaceutical composition comprising the compound according to claim 1 or 2 and a pharmaceutically acceptable carrier.

**5.** A chymase inhibitor comprising the compound according to claim 1 as an effective component.

6. The chymase inhibitor according to claim 5 wherein the effective component is at least one selected from the group consisting of 8-(4-chlorobenzyloxy)-5-(4-chlorobenzylsulfinyl) imidazo[1,2-d][1.2.4]triazine, 2-ethoxycarbonylmethyl-4-(4-chlorobenzylsulfinyl)-8-methylimidazo[1,5-d][1.2.4]triazine-1 (2H)-one, 8-(3-chlorobenzyloxy)-5-(3-chlorobenzylsulfinyl) imidazo[1,2-d][1.2.4]triazine, 2-(4-chlorobenzyl)-4-(4-chlorobenzylsulfinyl)-8-methylimidazo[1,5-d][1.2.4]triazine-1 (2H)-one, 5-(4-chlorobenzylsulfinyl)-8-ethoxycarbonylmethyloxyimidazo[1,2-d][1.2.4]triazine, 5-(4-chlorobenzylsulfinyl)-8-hydroxyimidazo[1,2-d][1.2.4]triazine, 1-(4-chlorobenzyloxy)-4-(4-chlorobenzylsulfinyl)-8-methylimidazo [1,5-d][1.2.4]triazine,4-(4-chlorobenzylsulfinyl)-1-(ethoxycarbonylmethyloxy)-8-methylimidazo[1,5-d][1.2.4]triazine, 8-(4-methylbenzyloxy)-5-(4-methylbenzylsulfinyl)imidazo[1,2-d] [1.2.4]triazine, 8-hydroxy-5-(3-phenylpropylsulfinyl)imidazo [1,2-d][1.2.4]triazine, 8-pentyloxy-5-pentylsulfinylimidazo[1, 2-d][1.2.4]triazine, 4-(4-chlorobenzylsulfinyl)-1-hydroxypyrrolo [1,2-d][1.2.4]triazine, 1-(4-chlorobenzyloxy)-4-(4-chlorobenzylsulfinyl)pyrrolo[1,2-d][1.2.4]triazine, 4-(4-chlorobenzylsulfinyl)-1-hydroxy-8-methylimidazo[1,5-d][1.2.4] triazine, 8-phenethyloxy-5-phenethylsulfinylimidazo[1,2-d] [1.2.4]triazine and 8-(3-phenylpropyloxy)-5-(3-phenylpropylsulfinyl)imidazo[1,2-d][1.2.4]triazine.

7. The chymase inhibitor according to claim 5 or 6, which is useful for the prevention and/or treatment of bronchial asthma, allergic rhinitis or hives.

8. A nitric oxide production inhibitor comprising the compound according to claim 1 as an effective component.

9. The nitric oxide production inhibitor according to claim 8 wherein the effective component is at least one selected from the group consisting of 4-(4-chlorobenzylsulfinyl)-1-hydroxypyrrolo[1,2-d][1.2.4]triazine, 1-ethoxy-4-ethylsulfinyl-8-methylimidazo[1,5-d][1.2.4]triazine, 8-ethoxy-5-ethylsulfinylimidazo[1,2-d][1.2.4]triazine, 5-(4-chlorobenzylsulfinyl)-8-hydroxyimidazo[1,2-d][1.2.4]triazine, 1-(4-chlorobenzyloxy)-4-(4-chlorobenzylsulfinyl)-8-methylimidazo[1,5-d][1.2.4]triazine, 2-(4-chlorobenzyl)-4-(4-chlorobenzylsulfinyl)-8-methylimidazo[1,5-d][1.2.4]triazine-1(2H)-one, 2-ethyl-4-ethylsulfinyl-8-methylimidazo[1,5-d][1.2.4]triazine-1(2H)-one and 2-ethoxycarbonylmethyl-4-(4-chlorobenzylsulfinyl)-8-methylimidazo[1,5-d][1.2.4]triazine-1(2H)-one.

10. The nitric oxide production inhibitor according to claim 8 or 9, which is useful for the prevention and/or treatment of cardiopathy and cerebrovascular disease, ishemic heart disease, septic shock, ache, rheumatic fever, arthritis, asthma, immunodeficiency, viral or non-viral infections, autoimmune diseases or cancer.

European Patent Office

# EUROPEAN SEARCH REPORT

Application Number
EP 95 11 8477

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | US-A-4 115 572 (R.PAUL ET AL) 19 September 1978<br>see whole document, especially column 2, lines 11-16<br>--- | 1-7 | C07D487/04<br>A61K31/53<br>//(C07D487/04, 253:00, 235:00), (C07D487/04, 253:00,209:00) |
| Y | EP-A-0 586 344 (CIBA-GEIGY AG) 9 March 1994<br>see whole text, especially page 3, lines 1-25<br>--- | 1-7 | |
| Y | EP-A-0 466 125 (YOSHITOMI PHARMACEUTICAL INDUSTRIES LTD.) 15 January 1992<br>see whole document, especially page 1, lines 1-7 and lines 42-46<br>--- | 1-7 | |
| Y | EP-A-0 057 289 (AMERICAN CYANAMID COMPANY) 11 August 1982<br>see whole document, especially page 3, lines 24-end of page.<br>--- | 1-7 | |
| A | J.HYPERTENSION, vol. 11, 1993<br>pages 1155-1159, XP 000565789<br>A HUSAIN 'The chymase-angiotensin system in humans'<br>----- | 1-10 | TECHNICAL FIELDS SEARCHED (Int.Cl.6)<br>C07D<br>A61K |

The present search report has been drawn up for all claims

| Place of search | Date of completion of the search | Examiner |
|---|---|---|
| MUNICH | 15 March 1996 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)